# EUROPÄISCHE PATENTSCHRIFT

(11) **EP 1 861 495 B1**
(45) Veröffentlichungstag und Bekanntmachung des Hinweises auf die Patenterteilung: **18.09.2013**
(21) Anmeldenummer: 06723146.4
(22) Anmeldetag: 28.02.2006
(51) Int. Cl.: C12N 15/10, C12Q 1/68, C12N 9/22

(54) **VERWENDUNG VON NUKLEASEN ZUM ABBAU VON NUKLEINSÄURE IN GEGENWART VON CHAOTROPEN AGENZIEN UND/ODER TENSIDEN**
USE OF NUCLEASES FOR DEGRADING NUCLEIC ACIDS IN THE PRESENCE OF CHAOTROPIC AGENTS AND/OR SURFACTANTS
UTILISATION DE NUCLÉASES POUR LA DÉCOMPOSITION D'ACIDES NUCLÉIQUES EN PRÉSENCE D'AGENTS CHAOTROPES ET/OU DE TENSIO-ACTIFS

(30) Priorität: 02.03.2005 DE 102005009479
(43) Veröffentlichungstag der Anmeldung: 05.12.2007
(73) Patentinhaber: Molzym GmbH & Co. KG, 28359 Bremen (DE)
(72) Erfinder: LORENZ, Michael, G., 26160 Bad Zwischenahn (DE)
(74) Vertreter: UEXKÜLL & STOLBERG
(86) Internationale Anmeldenummer: PCT/EP2006/001845
(87) Internationale Veröffentlichungsnummer: WO 2006/092278

(56) Entgegenhaltungen:
- CLAUDIA DISQUÉ, MICHAEL KALING, ULF MAGNUS WOLKERSDORFER, MICHAEL LORENZ: "Automatisierte Probenvorbereitung für die PCRDiagnostik von Sepsis" BIOSPEKTRUM, [Online] Nr. 1, 2006, Seiten 1-2, XP002384695 ISSN: 6 Gefunden im Internet: URL:http://www.molzym.com/pdf/IA_Fuji.pdf> [gefunden am 2006-06-08]
- Blood DNA removal and bacterial isolation kit MolYsis Plus5 - 5 ml blood protocol - retrieved 09.06.2006 from http://www.molzym.de/pdf/MolYsis_Plus5_010 6_Print.pdf 01-2006 XP002384962
- "OmniCleave? Endonuclease, Cat. Nos. OC7810K and OC7850K"[Online] Januar 2004 (2004-01), XP002384696 Gefunden im Internet: URL:http://www.epibio.com/pdftechlit/100pl 014.pdf> [gefunden am 2006-06-08]
- SHORTLE D: "GUANIDINE HYDROCHLORIDE DENATURATION STUDIES OF MUTANT FORMS OF STAPHYLOCOCCAL NUCLEASE" JOURNAL OF CELLULAR BIOCHEMISTRY, Bd. 30, Nr. 4, 1986, Seiten 281-290, XP002384697 ISSN: 0730-2312
- SAMBROOK, J., RUSSELL, D.: "Molecular Cloning - A laboratory manual third Edition" 2001, COLD SPRING HARBOUR LABORATORY PRESS , NEW YORK , XP002384968 in der Anmeldung erwähnt Seite 7.4 - Seite 7.8 Seite 7.85
- BOOM R ET AL: "RAPID AND SIMPLE METHOD FOR PURIFICATION OF NUCLEIC ACIDS" JOURNAL OF CLINICAL MICROBIOLOGY, WASHINGTON, DC, US, Bd. 28, Nr. 3, 1. März 1990 (1990-03-01), Seiten 495-503, XP000607196 ISSN: 0095-1137
- BURKE W F JR ET AL: "ISOLATION CHARACTERIZATION AND ACTIVATION OF THE MAGNESIUM DEPENDENT ENDO DNASE FROM BACILLUS-SUBTILIS" BIOCHEMISTRY, Bd. 16, Nr. 3, 1977, Seiten 403-410, XP002384698 ISSN: 0006-2960
- "ReadyPreps? Protein Preparation Kit Product Information Catalog No. RP78100" [Online] Januar 2006 (2006-01), , XP002384967 Gefunden im Internet: URL:http://www.ebiotrade.com/buyf/products f/epicentre/relation/readypreps.PDF> [gefunden am 2006-06-09] das ganze Dokument
- JEKEL M ET AL: "The periplasmic endonuclease I of Escherichia coli has amino-acid sequence homology to the extracellular DNases of Vibrio cholerae and Aeromonas hydrophila" GENE, ELSEVIER, AMSTERDAM, NL, Bd. 154, Nr. 1, 27. Februar 1995 (1995-02-27), Seiten 55-59, XP004042502 ISSN: 0378-1119
- SMITH K ET AL: "Comparison of commercial DNA extraction kits for extraction of bacterial genomic DNA from whole-blood samples." JOURNAL OF CLINICAL MICROBIOLOGY. JUN 2003, Bd. 41, Nr. 6, Juni 2003 (2003-06), Seiten 2440-2443, XP002384961 ISSN: 0095-1137
- PCR-Diagnosis of Blood Pathogens: Size Matters! retrieved from http://www.chemie.de/products/e/54806 found on 0806.2006 Chemie.DE Information Service GmbH XP002384699
- Directed isolation of bacterial DNA from whole blood Pre-analytic tool for the reliable diagnosis of sepsis by broad-range and multiplex PCR retrieved from http://www.molzym.com/home_e.htm found on 08.06.2006 XP002384700

### SEQUENZPROTOKOLL

<110> Molzym GmbH & Co. KG
   <120> Verwendung von Nukleasen zum Abbau von Nukleinsäure
   <130> P 70586
   <160> 6
<170> PatentIn version 3.1
<210> 1
   <211> 235
   <212> PRT
   <213> Escherichia coli
<220>
<210> 2
   <211> 230
   <212> PRT
   <213> Vibrio cholerae
<220>
   <223> Nuklease aus Vibrio cholerae
<400> 2
   Met Met Ile Phe Arg Phe Val Thr Thr Leu Ala Ala Ser Leu Pro Leu
   1 5 10 15
   Leu Thr Phe Ala Ala Pro Ile Ser Phe Ser His Ala Lys Asn Glu Ala
   20 25 30
   Val Lys Ile Tyr Arg Asp His Pro Val Ser Phe Tyr Cys Gly Cys Glu
   35 40 45
   Ile Pro Trp Gln Gly Lys Lys Gly Ile Pro Asp Leu Glu Ser Cys Gly
       50                  55                  60
   Tyr Gln Val Arg Lys Asn Glu Asn Arg Ala Ser Arg Ile Glu Trp Glu
   65                  70                  75                  80
   His Val Val Pro Ala Trp Gln Phe Gly His Gln Leu Gln Cys Trp Gln
                   85                  90                  95
   Gln Gly Gly Arg Lys Asn Cys Thr Arg Thr Ser Pro Glu Phe Asn Gln
               100                 105                 110
   Met Glu Ala Asp Leu His Asn Leu Thr Pro Ala Ile Gly Glu Val Asn
           115                 120                 125
   Gly Asp Arg Ser Asn Phe Ser Phe Ser Gln Trp Asn Gly Val Asp Gly
       130                 135                 140
   Val Thr Tyr Gly Gln Cys Glu Met Gln Val Asn Phe Lys Glu Arg Thr
   145                 150                 155                 160
   Ala Met Ala Gln Ser Val Gln Arg Ala Ile Ala Arg Thr Tyr Leu Tyr
                   165                 170                 175
   Met Ser Glu Gln Tyr Gly Leu Arg Leu Ser Lys Ala Gln Thr Gln Leu
               180                 185                 190
   Met Gln Ala Trp Asn Asn Gln Tyr Pro Val Ser Glu Trp Glu Cys Val
           195                 200                 205
   Arg Asp Gln Arg Ile Glu Lys Val Gln Gly Asn Ser Asn Leu Leu Cys
       210                 215                 220
   Ala Cys Lys Cys Pro Asn
   225                 230
<210> 3
   <211> 266
   <212> PRT
   <213> Erwinia chrysanthemi
<220>
   <223> Nuklease aus Erwinia chrysanthemi
<400> 3
   Met Leu Arg Asn Leu Val Ile Phe Ala Val Leu Gly Ala Gly Leu Thr
   1               5                   10                  15
   Thr Leu Ala Ala Ala Gly Gln Asp Ile Asn Asn Phe Thr Gln Ala Lys
               20                  25                  30
   Ala Ala Ala Ala Lys Ile His Gln Asp Ala Pro Gly Thr Phe Tyr Cys
           35                  40                  45
   Gly Cys Lys Ile Asn Trp Gln Gly Lys Lys Gly Thr Pro Asp Leu Ala
       50                  55                  60
   Ser Cys Gly Tyr Gln Val Arg Lys Asp Ala Asn Arg Ala Ser Arg Ile
   65                  70                  75                  80
   Glu Trp Glu His Val Val Pro Ala Trp Gln Phe Gly His Gln Arg Gln
                   85                  90                  95
   Cys Trp Gln Asp Gly Gly Arg Lys Asn Cys Thr Lys Asp Asp Val Tyr
               100                 105                 110
   Arg Gln Ile Glu Thr Asp Leu His Asn Leu Gln Pro Ala Ile Gly Glu
           115                 120                 125
   Val Asn Gly Asp Arg Gly Asn Phe Met Tyr Ser Gln Trp Asn Gly Gly
       130                 135                 140
   Glu Arg Gln Tyr Gly Gln Cys Glu Met Lys Ile Asp Phe Lys Ser Gln
   145                 150                 155                 160
   Leu Ala Glu Pro Pro Glu Arg Ala Arg Gly Ala Ile Ala Arg Thr Tyr
                   165                 170                 175
   Phe Tyr Met Arg Asp Arg Tyr Asn Leu Asn Leu Ser Arg Gln Gln Thr
   180 185 190
   Gln Leu Phe Asp Ala Trp Asn Lys Gln Tyr Pro Ala Thr Thr Trp Glu
   195 200 205
   Cys Thr Arg Glu Lys Arg Ile Ala Ala Val Gln Gly Asn His Asn Pro
   210 215 220
   Tyr Val Gln Gln Ala Cys Ser Pro Asp Ala Ala Pro Tyr Tyr Asn Gly
   225 230 235 240
   Leu Ser Leu Ile Met Ile Ala Ala Val Ala Thr Val Ala Ala Arg Trp
   245 250 255
   Leu Thr Pro Ala Gly His Leu Pro Ser Asp
   260 265
<210> 4
   <211> 230
   <212> PRT
   <213> Aeromonas hydrophila
<220>
   <223> Nuklease aus Aeromonas hydrophila
<400> 4
   Met Phe Arg Pro Leu Leu Ser Leu Cys Leu Ala Leu Leu Val Ser Ala
   1 5 10 15
   Pro Ala His Ala Asp Asn Ile Gln Thr Phe Arg Ala Ala Lys Gln Asp
   20 25 30
   Leu Asn Lys Leu Tyr Gln Ser His Pro Val Thr Phe Tyr Cys Gly Cys
   35 40 45
   Asn Ile Lys Phe Ser Gly Lys Lys Met Ala Pro Asp Trp Glu Ser Cys
   50 55 60
   Gly Tyr Leu Pro Gly Lys Gln Ala Glu Arg Ala Ser Arg Ile Glu Trp
   65 70 75 80
   Glu His Val Val Pro Ala Trp Glu Phe Gly His Gln Leu Gln Cys Trp
   85 90 95
   Gln Asp Gly Gly Arg Lys Asn Cys Gly Lys Ser Asp Glu Phe Asn Arg
   100 105 110
   Met Glu Gly Asp Met His Asn Leu Phe Pro Ala Ile Gly Glu Val Asn
   115 120 125
   Gly Asp Arg Ala Asn Phe Arg Phe Ser Asp Trp Asn Gly Lys Pro Asn
   130 135 140
   Gln Tyr Gly Lys Cys Gln Met Leu Val Asp Phe Lys Glu Arg Gln Val
   145 150 155 160
   Gln Pro Pro Lys Gly Pro Val Arg Gly Gln Ile Ala Arg Ala Tyr Leu
   165 170 175
   Tyr Met Ser Gln Gln Tyr Gly Leu Arg Leu Ala Ala Gln Gln Arg Lys
   180 185 190
   Leu Tyr Glu Ala Trp Asp Arg Gln Tyr Pro Ala Asp Arg Trp Glu Cys
   195 200 205
   Glu Arg Asn Arg Arg Ile Gly Lys Leu Gln Gly Asn Thr Asn Pro Phe
   210 215 220
   Ile Glu Lys Gln Cys Gln
   225 230
<210> 5
   <211> 17
   <212> DNA
   <213> Artificial
<220>
   <223> PCR-Primer 341f
<400> 5
   cctacgggag gcagcag 17
<210> 6
   <211> 17
   <212> DNA
   <213> Artificial
<220>
   <223> PCR-Primer 985r
<400> 6
   gtaaggttct tcgcgtt 17

## Beschreibung

Die Erfindung betrifft die Verwendung von Nukleasen, insbesondere von DNA-abbauenden Nukleasen, zum Abbau von Nukleinsäure in Gegenwart von einem oder mehreren chaotropen Agenzien und/oder einem oder mehreren Tensiden. Die vorliegende Erfindung betrifft ferner ein Verfahren zur Aufreinigung von RNA aus Mischungen aus DNA und RNA. Die Erfindung betrifft ferner ein Verfahren zur gezielten Isolierung von Nukleinsäuren aus mikrobiellen Zellen, die in einer Mischprobe vorliegen, die ferner höhere eukaryontische Zellen umfasst.

Die Isolierung und Reinigung von Nukleinsäuren aus biologischen Materialien stellt ein grundlegendes Verfahren im Rahmen der Probenvorbereitung für die molekularbiologische Analytik und human- oder veterinärmedizinische Diagnostik dar. So ist es beispielsweise wünschenswert, Krankheitsveranlagungen oder Infektionen schnell und zuverlässig nachzuweisen, um entsprechende Therapien einleiten zu können. Aber auch in anderen Anwendungsbereichen wie bei der Tier- und Pflanzenzucht, dem Pflanzenschutz, der Forensik, der Biotechnologie und der molekularbiologischen Forschung kommt den molekularbiologischen Analyse- und Diagnoseverfahren ein hoher Stellenwert zu.

Üblicherweise werden Nukleinsäuren im Stand der Technik mit Hilfe stark denaturierender und reduzierender Agenzien aus Zellen und Geweben freigesetzt. Dabei kommen regelmäßig chaotrope Agenzien, wie beispielsweise Guanidinsalze zur Anwendung. Diese stark denaturierend wirkenden Salze führen einerseits zum Zellaufschluss und sorgen anderseits dafür, dass Enzyme, die zum Abbau der aus den Zellen freigesetzten Nukleinsäuren führen würden, inaktiviert werden. Nachteil der Verwendung chaotroper Agenzien zum Zellaufschluss ist die Tatsache, dass weitere enzymatische Verfahrensschritte nur nach Entfernen dieser Agenzien aus dem Ansatz erfolgen können. So müssen beispielsweise bei einer RNA-Reiniung, die unter Verwendung chaotroper Salze zum Zellaufschluss durchgeführt wird, diese chaotropen Salze zunächst entfernt werden, bevor kontaminierende DNA durch Einwirkung von pankreatischer DNase I enzymatisch abgebaut werden kann. Diese Vorgehensweise ist jedoch mit einem erhöhten Zeit- und Materialaufwand verbunden, da mindestens ein weiterer Verfahrensschritt (z.B eine Filtration) durchgeführt werden muss.

Die freigesetzten Nukleinsäuren können anschließend durch entsprechende Methoden von anderen Zellbestandteilen befreit und aufgereinigt werden. Die freigesetzten Nukleinsäuren können beispielsweise durch Phenol-Chloroform-Extraktion (Sambrook, J., Russell, D.W. (2001): Molecular cloning - a laboratory manual. Cold Spring Harbor Laboratory Press, Cold Spring Harbor, New York) oder durch selektive Bindung an ein geeignetes Trägermaterial gereinigt werden. Als Trägermaterialien haben sich in den vergangenen Jahren insbesondere mineralische Trägermaterialien, wie beispielsweise gemahlenes Glaspulver, Diatomeenerde oder Silikamaterialien bewährt, wobei auch chemisch modifizierte Materialien wie Silikakarbid (U.S. Pat. No. 6,291,248) verwendet werden können. Kits zur Aufreinigung von Nukleinsäuren sind gegenwärtig von verschiedenen Firmen erhältlich, z.B. von der Molzym GmbH & Co KG (Bremen, DE), Qiagen (Hilden, DE), Macherey-Nagel (Düren, DE) oder Sigma (Deisenhofen, DE). Alle diese Verfahren gewährleisten in der Regel einen Reinheitsgrad der Nukleinsäuren, der sich für nachfolgende analytische und diagnostische Verfahren als hinreichend erweist.

Sambrook, J., Russell, D.W. (2001): Molecular cloning - a laboratory manual, 3rd edition, Cold Spring Harbor Laboratory Press, Cold Spring Harbor, New York, offenbaren ein Standardprotokoll für die RNA-Reinigung, bei dem Nukleasen durch Guanidinthiocyanat und Natriumlaurylsarcosinat inaktiviert werden (siehe Abschnitte 7.4-7.8).

Ein erheblicher Teil der molekularbiologischen Diagnostik verschiedener Krankheitserreger (wie beispielsweise der Nachweis pathogener Bakterien, Hefen, Pilze, Protozoen oder Viren) wird mittlerweile in der klinischen Praxis durch Verfahren bewältigt, die auf der Amplifikation von Nukleinsäuren (DNA oder RNA) des jeweiligen Erregers basieren. Zu diesem Zweck werden PCR-Primer für den Nachweis von Art- und Stamm-spezifischen Nukleotidsequenzen des Erregers verwendet. Diese spezifischen Nukleotidsequenzen können beispielsweise bei Bakterien IS-Elemente, Pathogenitätsfaktoren, speziesspezifische genomische Sequenzen oder auch bestimmte, weniger konservierte (gattungs- oder speziesspezifische) Bereiche auf dem bakteriellen 16S- oder 23S rRNA-Gen umfassen. Gegenwärtig werden zahlreiche bakterielle Erreger, insbesondere Erreger, die mittels herkömmlicher Kultivierungsverfahren nur unzureichend oder auf äußerst aufwendige Weise kultiviert werden können, in der klinischen Praxis durch Amplifikationsverfahren anhand stark konservierter Bereiche im Gen der 16S-rRNA identifziert. Zu diesen Erregern zählen u.a. verschiedene Arten der Gattungen Mycobacterium, Enterococcus, Streptococcus, Staphylococcus, Salmonella, Legionella, Clamydia und Shigella.

Die zum Nachweis verwendeten Nukleinsäure-Amplifikationsverfahren sind jedoch oftmals äußerst störanfällig, da die nachzuweisenden Erreger in dem untersuchten Probenmaterial in der Regel in einem Gemisch mit verschiedenen anderen Zelltypen (wie z.B. Blut- oder Gewebezellen des Patienten) vorliegen. Nukleinsäure-Präparationen, die aus solchen Mischproben gewonnen werden, enthalten normalerweise ein Gemisch von Nukleinsäuren aus allen Zelltypen, die in der Mischprobe vorhanden waren. Dies kann dazu führen, dass bei Verwendung solcher Nukleinsäure-Präparationen in den zum Zwecke der Erreger-Diagnostik durchgeführten Amplifikations-Verfahren unspezifische Amplifikationsprodukte entstehen, die die molekulare Diagnostik erschweren oder sogar vereiteln können. So lassen sich beispielsweise in Nukleinsäure-Präparationen aus Mischproben, die neben bakteriellen Zellen auch humane Gewebezellen enthielten, aufgrund der gemeinsamen Evolutionsgeschichte von eukaryontischen Zellorganellen und dem Bakterien-Genom unspezifische PCR-Produkte beobachten, wenn spezifische Primer für das Gen der 16S-rRNA verwendet werden.

Probleme dieser Art treten auch bei Nachweisverfahren mit Nukleinsäuren aus anderen Mischproben mit verschiedenen Arten von Zellen auf, z.B. bei Nukleinsäure-Präparationen aus Proben, die von pflanzlichen Organismen gewonnen wurden und neben einem nachzuweisenden, pflanzenpathogenen Bakterium in der Regel auch pflanzliche Zellen und Gewebe enthalten.

Der Nachweis von Erregern über Amplifikation spezifischer Nukleinsäuren ist jedoch für die klinische Diagnostik von großem Vorteil, da auch Erreger, die sich durch übliche Kultivierungsverfahren nicht identifizieren lassen, durch Sequenzanalyse und Datenvergleich mit Genbankeinträgen bestimmt werden können. Dies ist insbesondere bei der Diagnose der Sepsis von Bedeutung, da zahlreiche Blutbesiedler, die diesen Erkrankungszustand verursachen können, nicht durch das Anlegen von Blutkulturen nachgewiesen werden können.

Aufgabe der vorliegenden Erfindung ist es deshalb, Verfahren zur Verfügung zu stellen, mit denen man selektiv Nukleinsäure aus mikrobiellen Zellen isolieren kann, die in Mischproben mit höheren eukaryontischen Zellen bzw. Gewebe, vorliegen. Ferner ist es Aufgabe der vorliegenden Erfindung, Verfahren zur Verfügung zu stellen, mit denen man selektiv Nukleinsäure aus Blutprodukten, insbesondere Thrombozytenkonzentraten und Erythrozytenkonzentraten, oder aus anderen Körperflüssigkeiten, insbesondere Punktaten (Z.B. Gelenk, Augen), Liquor und Bronchial-Alveolar-Lavage, isolieren kann. Ferner ist es Aufgabe der vorliegenden Erfindung, Mittel bereitzustellen, mit deren Hilfe Nukleinsäuren in einem chaotropen Milieu abgebaut werden können. Erfindungsgemäß werden die obigen Aufgaben durch den Gegenstand der vorliegenden Ansprüche gelöst.

### Beschreibung der Figuren:

- Fig.1:: Elektrophoretische Trennung von extrahierter DNA; A: DNA stammt aus Extraktion mittels eines handelsüblichen Kits zur Nukleinsäure-Reinigung (Prinzip der Gesamtextraktion); B: DNA stammt aus Extraktion mittels des erfindungsgemäßen Verfahrens a: Reinkultur; b: unspiked Vollblut; c: spiked Vollblut.
- Fig.2:: A: PCR-Amplifikation des 16s-rRNA-Gens mit DNA aus Mischproben-Extrakten; A: DNA stammt aus Extraktion mittels eines handelsüblichen Kits zur Nukleinsäure-Reinigung (Prinzip der Gesamtextraktion); B: DNA stammt aus Extraktion mittels des erfindungsgemäßen Verfahrens. a: unspiked Vollblut; b: spiked Vollblut; c: Reinkultur.
- Fig.3:: Test (Doppelansätze) auf Rnase-Aktivität der Endonklease I aus E.coli mit Hefe-RNA (50 µg/Test, 16 h Inkubation). A: Kontroll-RNA ohne Inkubation; B,C: RNA ohne bzw. mit 2 Units Endonuklease I aus E. coli (37°C); D, E: wie B/C aber bei 55°C.
- Fig.4:: Abhängigkeit der enzymatischen Aktivität der Endonuklease I aus E.coli von der Magnesiumchlorid-Konzentration.
- Fig.5:: Abhängigkeit der enzymatischen Aktivität der Endonu-klease I aus E.coli vom pH-Wert.
- Fig.6:: Abhängigkeit der enzymatischen Aktivität der Endonu-klease I von der Temperatur. Endonuklease I ist ein thermophiles Enzym (Optimum bei 65 °C).
- Fig.7:: Inkubation von Kalbsthymus-DNA (5 µg) mit 200 Units Endonuklease I bzw. 1000 U pankreatischer DNase I in Anwesenheit von 1 mol/l Guanidin-HCl (Bahnen 1,3) bzw. 1 mol/l Guanidinisothiocyanat (Bahnen 2,4). Zu erkennen ist, dass Endonuklease I unter beiden Bedingungen (Bahnen 1,2) die DNA vollständig abbaut, während die DNase I nur noch geringe Aktivität (Bahn 3, siehe Banden bei 0,5 kb) bzw. gar keine Aktivität mehr besitzt (Bahn 4).

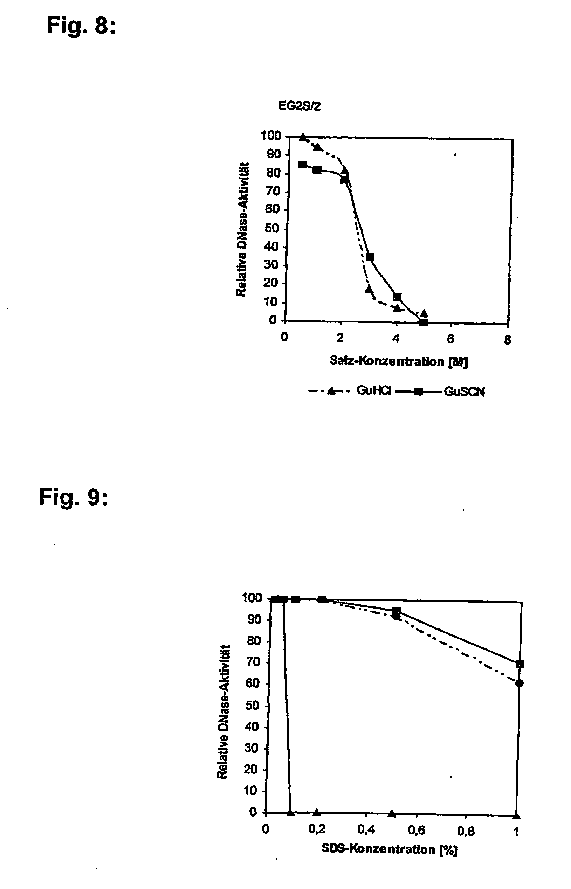

- Fig.8:: Guanidinsalz-Resistenz der DNase von EG2S/2 (Halobacillus sp.). Die Aktivität des Enzyms liegt bei 2 M Guanidinhydrochlorid und -Isothiocyanat noch bei ca. 80 % der Aktivität ohne Guanidinsalze. 80 % der DNase-Aktivität von SJ1/4 war sogar noch bei 4 M Guanidin-Isothiocyanat bzw. 5 M Guanidinhydrochlorid vorhanden. 100% entspricht ca. 30 Units Enzym im Assay. GuHCl (Guanidinhydrochlorid), GuSCN (Guanidin-Isothiocyanat).
- Fig.9:: DNase Aktivitäten von SJ1/4 (Kreise), EG2S/2 (Vierecke) und Merck Benzonase (Dreiecke) in Anwesenheit von SDS. 100% entspricht ca. 30 Units Enzym. Klar zu erkennen ist die Empfindlichkeit der kommerziellen Benzonase, während die Isolate noch 60-70% Aktivität bei 1 % SDS besitzen.

Es wurde nunmehr überraschenderweise herausgefunden, dass die Endonuklease I aus *Escherichia coli* (SEQ ID NO:1), *Vibrio cholerae* (SEQ ID NO:2), *Erwinia chrysanthemi* (SEQ ID NO:3) *und Aeromonas hydrophila* (SEQ ID NO:4) unter chaotropen Umgebungsbedingungen, die zur Lyse von Zellen führen, nicht denaturiert werden und somit ihre enzymatische Aktivität beibehalten. Somit eignen sich diese Nukleasen insbesondere für den Abbau von DNA in Gegenwart von chaotropen Agenzien und/oder Tensiden. Die Erfindung betrifft somit gemäß eines ersten Aspektes die Verwendung einer DNA-abbauenden Nuklease, die keine RNAse-Aktivität aufweist, zum Abbau von DNA in Gegenwart von einem oder mehreren chaotropen Agenzien und einem oder mehreren Tensiden, dadurch gekennzeichnet, dass die DNA-abbauende Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

In einer bevorzugten Ausführungsform der Erfindung
a) weist die DNA-abbauende Nuklease die in SEQ ID NO:1 gezeigte Aminosäuresequenz auf;
b) weist die DNA-abbauende Nuklease eine Aminosäuresequenz auf, bei der bis zu 40% der Aminosäuren von der in SEQ ID NO:1 gezeigten Aminosäuresequenz abweichen; oder
c) ist die DNA-abbauende Nuklease ein enzymatisch aktives Fragment von (a) oder (b).

Im Zusammenhang mit der vorliegenden Erfindung bezeichnet der Begriff "Nukleinsäure" einzelsträngige, doppelsträngige und teilweise doppelsträngige Ribonukleinsäuren (RNA) und Desoxyribonukleinsäuren (DNA). Des weiteren umfasst der Begriff Duplex- und Triplex-Strukturen, die aus DNA und/oder RNA aufgebaut sein können.

Der Begriff "Nuklease" bezeichnet im Rahmen der vorliegenden Erfindung ein Enzym, welches die Esterbindung zwischen der 5'-Phosphatgruppe eines Nukleotids und der 3'-Hydroxylgruppe des benachbarten Nukleotids in einer Nukleinsäure hydrolytisch spaltet und damit die Degradation eines DNA- oder RNA-Moleküls bewirkt. Nukleasen sind aus zahlreichen Organismen bekannt. Nukleasen spalten entweder RNA- oder DNA-Moleküle zu kleineren Einheiten oder sogar in ihre Monomere. Es sind darüber hinaus auch Enzyme bekannt, die beide Aktivitäten aufweisen. Als "DNA-abbauende Nuklease" wird folglich eine Nuklease verstanden, die in der Lage ist, einzelsträngige, doppelsträngige und teilweise doppelsträngige DNA-Moleküle in kleinere Einheiten oder Monomere zu spalten. Solche Enzyme werden im Stand der Technik auch als "DNasen" bezeichnet.

Bei der Nuklease oder DNA-abbauenden Nuklease kann es sich erfindungsgemäß um eine Endonuklease oder um eine Exonuklease handeln. Als Exonuklease wird vorliegend ein Enzym bezeichnet, welches eine Nukleinsäurekette ausgehend von einem oder von beiden Enden der Kette abbaut. Bei einem durch Endonukleasen vermittelten Abbau einer Nukleinsäurekette hingegen erfolgt dieser Abbau ausgehend von einer Stelle innerhalb der Kette. Es ist bevorzugt, dass es sich bei der Nuklease um eine Endonuklease handelt.

Bei der Endonuklease, die erfindungsgemäß unter chaotropen Bedingungen verwendet wird, kann es sich beispielsweise um die Endonuklease I (EndA) aus *Escherichia coli* handeln (Jekel, M., Wackernagel, W., J. Bacteriol. 176:1550, 1994). Dieses Enzym ist von verschiedenen Anbietern, z.B. von der Molzym GmbH & Co KG (Bremen Deutschland) zu erhalten. Ferner kann es sich um die in SEQ ID NO:2 gezeigte Nuklease aus *Vibrio cholerae* handeln (Focareta T., Manning P.A.; Gene 53(1):31-40(1987)). Auch die in SEQ ID NO:3 gezeigte Nuklease aus *Erwinia chrysanthemi* (Moulard M., Condemine G., Robert-Baudouy J.; Mol. Microbiol. 8(4):685-695 (1993)) oder die in SEQ ID NO:4 gezeigte Nuklease aus *Aeromonas hydrophila* (Focareta T., Manning P.A.; Gene 53(1):31-40(1987); Chang M.-C., Chang S.-Y., Chen S.-L., Chuang S.-M.; Gene 122(1):175-180 (1992)) können im Rhamen der Erfindung verwendet werden. Gemäß einer besonders bevorzugten Ausführungsform handelt es sich bei der Endonuklease um das in SEQ ID NO:1 gezeigte Polypeptid aus *Escherichia coli.* Es wurde im Rahmen der vorliegenden Erfindung überraschenderweise festgestellt, das die obigen Enzyme unter chaotropen Bedingungen, d.h. in Gegenwart von chaotropen Agenzien und/oder Tensiden, in der Lage sind, wirkungsvoll DNA zu degradieren. Unter diesen Bedingungen werden herkömmliche, im Stand der Technik bekannte Enzyme wie pankreatische DNase I oder Nuklease aus der bakterien-Gattung *Serratia* (z.B. Benzonase, Merck) inaktiviert.

Erfindungsgemäß sind Varianten und Derivate der in SEQ ID NO:1, SEQ ID NO:2, SEQ ID NO:3 und SEQ ID NO:4 gezeigten Polypeptide sowie enzymatisch aktive Fragmente der Polypeptide und ihrer Varianten ebenfalls eingeschlossen.

Unter Varianten eines Polypeptids werden solche Peptide oder Polypeptide verstanden, die sich durch einen oder mehrere Austausche von Aminosäuren von der Aminosäuresequenz des in SEQ ID NO:1, SEQ ID NO:2, SEQ ID NO:3 oder SEQ ID NO:4 gezeigten Polypeptids unterscheiden.

Dabei kann grundsätzlich jeder Aminosäurerest der in den SEQ ID NO:1 bis SEQ ID NO:4 gezeigten Aminosäuresequenzen gegen eine abweichende Aminosäure ausgetauscht sein, sofern es sich bei der resultierenden Sequenz der Variante weiterhin um ein enzymatisch aktives Polypeptid mit Nuklease-Funktion handelt. Umfasst sind insbesondere solche Varianten, bei denen insgesamt bis zu 5 %, 10%, 15%, 20%, 30%, oder 40% der Aminosäuren von der in SEQ ID NO:1 gezeigten Aminosäuresequenz abweichen. Als Varianten gelten ferner auch solche Polypeptide, bei denen eine oder mehrere Aminosäuren in die Aminosäuresequenz des in SEQ ID NO:1, SEQ ID NO:2, SEQ ID NO:3 oder SEQ ID NO:4 gezeigten Polypeptids eingefügt wurden. Solche Insertionen können an jeder Position des in SEQ ID NO:1 SEQ ID NO:2, SEQ ID NO:3 oder SEQ ID NO:4 gezeigten Polypeptids erfolgen. Ferner gelten auch Polypeptide als Varianten des in SEQ ID NO:1, SEQ ID NO:2, SEQ ID NO:3 oder SEQ ID NO:4 gezeigten Polypeptide, bei denen eine oder mehrere Aminosäuren im Vergleich zu dem in SEQ ID NO:1, SEQ ID NO:2, SEQ ID NO:3 oder SEQ ID NO:4 gezeigten Polypeptid fehlen. Solche Deletionen können jede Aminosäure-Position der Sequenz von SEQ ID NO:1, SEQ ID NO:2, SEQ ID NO:3 oder SEQ ID NO:4 betreffen. Unter enzymatisch aktiven Fragmenten der in SEQ ID NO:1, SEQ ID NO:2, SEQ ID NO:3 oder SEQ ID NO:4 gezeigten Sequenz oder deren Varianten werden solche Peptide oder Polypeptide verstanden, die sich von der in SEQ ID NO:1, SEQ ID NO:2, SEQ ID NO:3 oder SEQ ID NO:4 gezeigten Aminosäuresequenz oder deren wie oben definierten Varianten durch das Fehlen einer oder mehrerer Aminosäuren am N-Terminus und/oder am C-Terminus des Peptids oder Polypeptids unterscheiden. Derivate der in SEQ ID NO:1, SEQ ID NO:2, SEQ ID NO:3 und SEQ ID NO:4 gezeigten Polypeptide oder der Varianten desselben bezeichnen vorliegend Polypeptide, die gegenüber einem in SEQ ID NO:1, SEQ ID NO:2, SEQ ID NO:3 oder SEQ ID NO:4 gezeigten Polypeptid oder seiner Varianten strukturelle Modifikationen, wie beispielsweise modifizierte Aminosäuren, aufweisen.

Bei diesen modifizierten Aminosäuren kann es sich erfindungsgemäß um Aminosäuren handeln, die durch Phosphorylierung, Glykosylierung, Acetylierung, Thiolierung, Verzweigung und/oder Zyklisierung verändert wurden. Es wird bevorzugt, dass die Varianten oder Derivate der in SEQ ID NO:1, SEQ ID NO:2, SEQ ID NO:3 und SEQ ID NO:4 gezeigten Polypeptids bzw. die aktiven Fragmente dieses Polypeptids oder seiner Varianten 75 %, vorzugsweise bis zu 80%, 85%, 90% oder sogar bis zu 99% der Aktivität des in SEQ ID NO:1, SEQ ID NO:2, SEQ ID NO:3 oder SEQ ID NO:4 gezeigten Polypeptids aufweisen.

Unter chaotropen Agenzien werden im Rahmen der vorliegenden Erfindung Substanzen verstanden, die regelmäßige molekulare Strukturen, welche auf der Bildung von Wasserstoff-Brückenbindungen beruhen, zerstören. Chaotrope Stoffe destabilisieren die Konformation von Makromolekülen, indem sie die Bildung der zur Solvatation notwendigen H₂O-Käfigstrukturen verhindern. Chaotrope Salze weisen eine hohe Affinität zu Wasser auf und bilden daher eine große Hydrathülle aus. Chaotropen Agenzien (wie chaotrope Salze) sind dem Fachmann hinlänglich bekannt (siehe beispielsweise Guanidinhydrochlorid, Guanidinisothiocyanat, Natriumperchlorat, Natriumiodid, Trichloracetat, Harnstoff, Rhodanitsalz. Bei den chaotropen Agenzien, in deren Gegenwart die erfindungsgemäße Nuklease verwendet werden kann, zählen insbesondere chaotrope Salze, wie beispielsweise Guanidin-Hydrochlorid, Natriumiodid, Guanidin-Isothiocyanat oder Mischungen derselben.

Ferner kann die erfindungsgemäße Nuklease auch in Gegenwart von einem oder mehreren Tensiden zum Abbau von Nukleinsäuren verwendet werden. Als "Tenside" werden vorliegend grenzflächenaktive Stoffe bezeichnet, welche sich bevorzugt in der Berührungsfläche zweier Medien konzentrieren, z.B. an der Grenzfläche Wasser/Luft. Erfindungsgemäß können anionische, kationische, amphotere oder nicht-ionische Tenside verwendet werden.

Anionische Tenside weisen eine negative Ladung des Moleküls auf und umfassen, z.B. Alkylbenzolsulfonate und Alkansulfonate. Kationische Tenside tragen eine positive Ladung und umfassen, z.B. Distearyldimethylammoniumchlorid. Nichtionische Tenside sind ungeladen und umfassen Fettalkoholethoxylate und Alkylpolyglucoside. Amphotere Tenside tragen sowohl eine positive als auch eine negative Ladung und umfassen beispielsweise Betaine. Tenside, in deren Gegenwart die Nuklease im Rahmen der vorliegenden Erfindung verwendet werden kann, umfassen beispielsweise Natrium-Dodecylsulfat (SDS), BRIJ 40, Tween-20, Desoxycholat, Triton X-100 oder Mischungen der genannten Tenside in verschiedenen Mischungsverhältnissen.

Im Rahmen der vorliegenden Erfindung ist es bevorzugt, dass die Gesamtkonzentration der chaotropen Agenzien im Ansatz von 0,1 mol/l bis 2,5 mol/l beträgt, wobei ein Bereich von 0,5 mol/l - 2 mol/l und ein Bereich von 1 mol/l - 1,5 mol/l insbesondere bevorzugt sind. Dies bedeutet, dass die Konzentration eines chaotropen Salzes wie Guanidinhydrochlorid oder Guanidinisothiocyanat bis zu 0,1 mol/l, 0,5 mol/l, 1 mol/l, 1,5 mol/l, 2 mol/l oder 2,5 mol/l betragen kann, sofern dieses Salz als einzige chaotrope Substanz im Ansatz verwendet wird. Werden Mischungen aus mehreren chaotropen Agenzien verwendet, wie beispielsweise Mischungen aus Guanidinhydrochlorid oder Guanidinisothiocyanat, so sollte die Gesamtkonzentration an chaotropen Agenzien erfindungsgemäß in den angegebenen Bereichen liegen.

Die Gesamtmenge an Tensid, die verwendet werden kann, ohne dass es zu einer Inaktivierung der Nuklease kommt, kann erfindungsgemäß bis zu 2 Gew.-% der gesamten Mischung betragen.. Gemäß einer bevorzugten Ausführungsform beträgt die Gesamtmenge an Tensid im Ansatz 0,1 - 2 Gew.-%, wobei Mengen von 0,5 - 1,5 Gew.-% und 1,2 - 1,4 Gew.-% besonders bevorzugt sind.

Es können selbstverständlich auch Mischungen von chaotropen Agenzien (wie chaotropen Salzen) und Tensiden verwendet werden. In diesen Fällen müssen Tensidmenge und Konzentration der chaotropen Substanz entsprechend aneinander angepasst sein, um noch eine Funktionstüchtigkeit der Nuklease zu gewährleisten. Der Fachmann kann mit Hilfe einfacher Versuche ausfindig machen, welche chaotrope(n) Substanz(en) in welcher Konzentration mit welchem (welchen) Tensid(en) gemeinsam verwendet werden kann (können).

Aufgrund der unerwarteten Eigenschaft, auch in Gegenwart chaotroper Agenzien Aktivität zu entfalten, können die erfindungsgemäßen Nukleasen auf zahlreichen technischen Gebieten vorteilhaft zur Anwendung gelangen. Beispielsweise lassen sich DNA-abbauende Nukleasen für die nachstehend beschriebene, gezielte Isolierung von Nukleinsäure aus Mischproben, die mikrobielle Zellen sowie höhere eukaryontische Zellen und/oder Gewebe umfassen, verwenden. Darüber hinaus lassen sich DNA-abbauenden Nukleasen gezielt für den Abbau kontaminierender DNA im Zuge von RNA-Isolierungen verwenden. Die Isolierung von RNA erfolgt regelmäßig unter chaotropen Bedingungen, wobei zum Entfernen von DNA eine aufwendige Umpufferung erfolgt. Diese Umpufferung ist bei Verwendung einer DNA-abbauenden Nuklease, die unter chaotropen Bedingungen stabil ist, entbehrlich. Somit lassen sich Material- und Zeitaufwand deutlich reduzieren.

Demnach betrifft die vorliegende Erfindung gemäß eines weiteren Aspektes ein Verfahren zur Isolierung und/oder Aufreinigung von RNA aus Mischungen, die RNA und DNA umfassen, bei dem man
a) die DNA unter Verwendung einer DNA-abbauenden Nuklease, die keine RNAse-Aktivität aufweist, in Gegenwart von einem oder mehreren chaotropen Agenzien und einem oder mehreren Tensiden abbaut; und
b) die RNA mittels geeigneter Verfahren isoliert bzw. aufreinigt,
dadurch gekennzeichnet, dass die DNA-abbauende Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

In einer bevorzugten Ausführungsform der Erfindung
a) weist die Endonuklease die in SEQ ID NO:1 gezeigte Aminosäuresequenz auf;
b) weist die Endonuklease eine Aminosäuresequenz auf, bei der bis zu 40% der Aminosäuren von der in SEQ ID NO:1 gezeigten Aminosäuresequenz abweichen; oder
c) ist die Endonuklease ein enzymatisch aktives Fragment von (a) oder (b).

Gemäß einer bevorzugten Ausführungsform der Erfindung handelt es sich bei den chaotropen Agenzien um chaotrope Salze. Vorzugsweise handelt es sich bei den chaotropen Salzen um Guanidinhydrochlorid, Guanidinisothiocyanat und/oder Natriumiodid. Gemäß einer weiteren Ausführungsform handelt es sich bei den Tensiden um Natriumdodecylsulfat, Brji40, Triton X-100 und/oder Tween20 .

Die Gesamtkonzentration der chaotropen Agenzien sowie die Gesamtmenge an Tensiden entsprechen den oben beschriebenen Konzentrationen und Mengen.

Die DNA-abbauende Nuklease kann Teil eines Puffers sein, der die entsprechenden chaotropen Agenzien und/oder Tenside umfasst, oder kann einem solchen Puffer im Verlauf der Reaktion zugesetzt werden, beispielsweise nach einem Zellaufschluss.

Die nach der Inkubation mit der DNA-abbauenden Nuklease im Ansatz verbliebene RNA kann nach herkömmlichen Verfahren aufgereinigt werden, beispielsweise unter Verwendung von Spin-Säulen, wie sie u.a. von der Firma Molzym (Bremen, DE) als PrestoSpin R erhältlich sind.

Die vorliegende Erfindung betrifft demgemäß ferner ein Verfahren zur gezielten Isolierung von Nukleinsäuren aus mikrobiellen Zellen, die in einer Mischprobe vorliegen, welche Zellen und/oder Gewebe höherer Eukaryonten umfasst, bei dem man
a) nur die höheren eukaryontischen Zellen und/oder Gewebe durch Zugabe eines oder mehrerer chaotroper Agenzien und eines oder mehrerer Tenside lysiert, wobei die mikrobiellen Zellen intakt bleiben;
b) die mikrobiellen Zellen von den lysierten höheren eukaryontischen Zellen und/oder Geweben abtrennt;
c) die mikrobiellen Zellen lysiert, sodass die Nukleinsäuren aus diesen freigesetzt werden;
d) die in Schritt c) freigesetzten Nukleinsäuren isoliert;
wobei man während oder nach Schritt a) eine DNA-abbauende Nuklease zusetzt, die keine RNAse-Aktivität aufweist, um die aus den höheren eukaryontischen Zellen freigesetzte DNA aus der Probe zu entfernen, dadurch gekennzeichnet, dass die DNA-abbauende Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

In einer bevorzugten Ausführungsform der Erfindung
a) weist die DNA-abbauende Nuklease die in SEQ ID NO:1 gezeigte Aminosäuresequenz auf;
b) weist die DNA-abbauende Nuklease eine Aminosäuresequenz auf, bei der bis zu 40% der Aminosäuren von der in SEQ ID NO:1 gezeigten Aminosäuresequenz abweichen; oder
c) ist die DNA-abbauende Nuklease ein enzymatisch aktives Fragment von (a) oder (b).

Die vorliegende Erfindung stellt ein Verfahren zur gezielten Isolierung von Nukleinsäure aus Mikroroganismen bereit. Die mikrobiellen Zellen können dabei in einem Gemisch (z.B. einer flüssigen Probe) vorliegen, die darüber hinaus Zellen und/oder Gewebe eukaryontischen Ursprungs enthält. Ferner ist das erfindungsgemäße Verfahren geeignet, Nukleinsäure aus Blutprodukten, insbesondere Thrombozytenkonzentraten und Erythrozytenkonzentraten, oder aus anderen Körperflüssigkeiten, insbesondere Punktaten (Z.B. Gelenk, Augen), Liquor und Bronchial-Alveolar-Lavage, zu isolieren. Im Rahmen der vorliegenden Erfindung wird unter dem Begriff "Verfahren zur gezielten Isolierung" ein Verfahren verstanden, welches es ermöglicht, aus einer solchen Mischprobe in reproduzierbarer Weise Nukleinsäuren verfügbar zu machen, die ausschließlich oder im überwiegenden Maße aus den in der Probe enthaltenden mikrobiellen Zellen stammt.

Vorzugsweise erfolgt durch das erfindungsgemäße Verfahren eine sequentielle Lyse der in der Probe befindlichen Zellen, wobei zunächst die höheren eukaryontischen Zellen und/oder Gewebe lysiert werden und in einem nachgeschalteten Schritt (nach Abtrennung von Zelltrümmern und Resten der lysierten höheren eukaryontischen Zellen und/oder Gewebe) der Aufschluss der (prokaryontischen oder eukaryontischen) mikrobiellen Zellen erfolgt. Die auf diese Weise bereitgestellte Nukleinsäure kann anschließend durch Bindung und Reinigung mittels herkömmlicher Verfahren isoliert werden.

Im Rahmen der vorliegenden Erfindung bezeichnen die Begriffe "mikrobielle Zelle" oder "Mikroorganismus" eine diverse Gruppe von Organismen, die in der Natur selbständig als einzelne Zelle oder als Zell-Cluster existieren, und sich damit von höheren eukaryontischen Zellen (wie beispielsweise von tierischen Zellen in einem Gewebe, z.B. Gewebezellen eines Säugetiers) unterscheiden, die in der Natur nicht als einzelne Zelle vorkommen, sondern auschließlich als Bestandteile mehrzelliger Organismen. Bei den mikrobiellen Zellen kann es sich erfindungsgemäß beispielsweise um prokaryontische Zellen, wie Bakterien oder Archaebakterien, oder auch um eukaryontische Zellen, wie Hefen, niedere und höhere Pilze oder Protozoen, handeln. Gemäß einer bevorzugten Ausführungsform handelt es sich bei den mikrobiellen Zellen um prokaryontische Zellen. Gemäß einer besonders bevorzugten Ausführungsform handelt es sich bei den prokaryontischen Zellen um Bakterien.

Im Rahmen der vorliegenden Erfindung wird unter dem Begriff "prokaryontische Zelle" oder "Prokaryont" jede Zelle bzw. jeder Organismus verstanden, die (der) zur phylogenetischen Gruppe der Archaea oder Bacteria gehört (vergleiche Balows, Trüper, Dworkin, Harder, Schleifer: The Procaryotes; Kapitel 142, Seiten 2696-2736 (1992).

Prokaryontische Zellen weisen deutliche Unterschiede zu eukaryontischen Zellen auf, die sich in strukturellen Merkmalen von Zellorganellen, Zellwand und ähnlichem widerspiegeln. Diese Charakteristika sind dem Fachmann hinlänglich bekannt. Somit umfassen die Begriffe "mikrobielle Zelle" oder "Mikroorganismus" insbesondere verschiedene Gattungen von gram-positiven und gram-negativen Bakterien, beispielsweise pathogene Bakterien der Gattungen *Mycobacterium, Enterococcus, Streptococcus, Staphylococcus, Salmonella, Legionella, Clamydia, Shigella, Pseudomonas, Listeria, Yersinia, Corynebacterium, Bordetella*, *Bacillus, Clostridium, Haemophilus, Helicobacter und Vibrio.*

Daneben kann es sich bei den mikrobiellen Zellen oder Mikroorganismen erfindungsgemäß auch um eukaryontische Zellen handeln. Gemäß einer bevorzugten Ausführungsform handelt es sich bei den mikrobiellen Zellen um Pilz-Zellen. Als Pilze, deren Nukleinsäuren sich gemäß des Verfahrens der vorliegenden Erfindung isolieren lassen, kommen insbesondere pathogene Pilze der Gattungen *Aspergillus* (z.B. *A. fumigatus*, *A*. *niger, A. flavus, A. nidulans*), *Basidiobolus* (z.B. *B. microsporus, B. ranarum*)*, Cephalosporium* (z.B. C. *chrysogenum, C*. *coremioides, C*. *diospyri, C*. *gregatum*) sowie andere pathogene Pilze der Gattungen *Entomophthora, Skopulariopsis, Mucor, Rhizomucor, Absidia, Rhizopus, Altenaria, Stemphylium*, *Botrytis, Chrysosporium, Curvularia, Helmithosporium, Hemispora, Nigrospora, Paecilomyces, Phoma*, *Thielavia oder Syncephalastrum* in Betracht. Erfindungsgemäß umfasst sind auch pathogene Hefen der Gattung *Candida*, z.B. *C*. *albicans, C*. *guilliermondii, C*. *kruzei*, *C*. *parapsilosis, C*. *tropicalis* und andere.

Ferner kann es sich bei den mikrobiellen Zellen oder Mikroorganismen erfindungsgemäß auch um Algen, wie beispielsweise *Ceratium massiliense, Dinophysis nitra, Gymnodinium sanguineum*, *Trachelomonas* spp., *Euglena spp., Coscinodiscus spp., Eremosphaera, Chlorella, Chlorococcum* oder Protozoen, wie beispielsweise *Cryptosporidium parvum, Cryptosporidium hominis,*

*Cryptosporidium serpentis, Toxoplasma gondii, Trypanosoma brucei, Trypanosoma cruzei, Plasmodium falciparum*, *Plasmodium malariae* handeln. Bei den Protozoen kann es sich insbesondere um deren Dauerformen handeln, die teilweise mantelartige Hüllen aus Proteoglycanen aufweisen.

Der Begriff "eukaryontische Zelle" oder "Eukaryont" bezeichnet vorliegend jede Zelle von ein- oder mehrzelligen Organismen, die zur phylogenetischen Gruppe der Eukarya gehört. Diese Zellen besitzen einen Zellkern, der von einer Zellmembran umschlossen ist und mehrere DNA-Moleküle umfasst, die sich über Mitose teilen. Eukaryontische Zellen umfassen einzellige Organismen wie einzellige Algen, Pilze (z.B. Hefen) oder Protozoen, die auch als Parasiten, Kommensalen oder Saprophyten zeitweise oder dauerhaft in oder auf einem Wirtsorganismus leben können. Als eukaryontische Zellen werden darüber hinaus auch die Zellen von mehrzelligen Organismen, wie beispielsweise von tierischen Organismen, wie Säugetieren, Pilzen oder Pflanzen verstanden.

Unter dem Begriff "höhere eukaryontische Zelle" wird im Rahmen der vorliegenden Erfindung eine eukaryontische Zelle höheren Entwicklungsgrades verstanden, wie sie beispielsweise in tierischen oder pflanzlichen Organismen vorkommen. Bei diesen Zellen kann es sich einerseits um Zellen handeln, die in einem Gewebe organisiert sind, d.h. die höhere eukaryontische Zelle führt nicht selbständig alle lebensnotwendige biochemischen und metabolischen Funktionen aus, sondern ist in der Regel durch Spezialisierung an die Ausführung einer oder mehrerer Funktionen angepasst.

Zum anderen umfasst der Begriff einzelne Zellen (z.B. Blutkörperchen, Spermatozoen), wie sie in einem Liquor eines Säugetieres (z.B. in Blut oder Lymphflüssigkeit) oder einem Ausscheidungsprodukt eines Säugetieres (z.B. in Urin oder Speichel) vorkommen.

Höhere eukaryontische Zellen sind u.a. alle Zellen aus einem Säugetier, einem Insekt, einem Weichtier oder aus einer höheren Pflanze (z.B. Einkeim- und Zweikeimblättrige). Als "höheres eukaryontisches Gewebe" wird im Rahmen der vorliegenden Erfindung demnach eine Ansammlung von höheren eukaryontischen Zellen verstanden, die in einem Zellverbund organisiert sind. Beispiele für höhere eukaryontische Gewebe sind beispielsweise die Organe der Säugetiere (Leber, Herz, Haut, Bauchspeicheldrüse) oder Blatt- oder Wurzel-Gewebe einer Pflanze. Gemäß einer bevorzugten Ausführungsform der Erfindung stammt das höhere eukaryontische Gewebe aus einem Säugetier. Gemäß einer besonders bevorzugten Ausführungsform der Erfindung stammt das höhere eukaryontische Gewebe aus einem Menschen.

Der Begriff "Mischprobe" bezeichnet im Zusammenhang mit der vorliegenden Erfindung eine Probe, die mindestens zwei verschiedene Nukleinsäure-enthaltende Zellen (wie z.B. Blut- oder Gewebezellen eines Säugetiers, Bakterienzellen, Pflanzengewebe-Zellen oder Pilzmycel-Zellen) umfasst. Vorzugsweise stammt die Mischprobe aus einer natürlichen Umgebung, wie beispielsweise aus einem menschlichen, pflanzlichen oder tierischen Organismus. Gemäß einer bevorzugten Ausführungsform handelt es sich bei der Mischprobe um eine Blutprobe. Gemäß einer besonders bevorzugten Ausführungsform handelt es sich bei der Mischprobe um eine Blutprobe eines Säugetiers, z.B. eines Menschen. Bei der Mischprobe kann es sich darüber hinaus auch um Blutprodukte, insbesondere Thrombozytenkonzentrate und Erythrozytenkonzentrate, andere Körperflüssigkeiten, insbesondere Punktate (Z.B. Gelenk, Augen), Liquor und Bronchial-Alveolar-Lavage, handeln.

Im Rahmen der vorliegenden Erfindung wird unter dem Begriff "Lyse" im Zusammenhang mit Zellen generell jeder Vorgang verstanden, der zur Zerstörung der äußeren Struktur der Zelle und deren Organellen führt. Die Lyse der Zelle führt zum Aufschluss der intakten Zelle sowie zur Freisetzung der Nukleinsäure aus dem jeweiligen Zellkompartiment bzw. Organell (Zellkern, Mitochondrien, Chloroplasten).

Die Lyse der Zellen erfolgt durch Zerstörung der Strukturen (wie beispielsweise Membranen und Zellwand), die die Nukleinsäuren der Zellen umschließen und vom umgebenden Medium trennen. So ist die Desoxyribonukleinsäure (DNA) bei eukaryontischen Zellen mindestens durch Kernhülle, Plasmamembran und ggf. Zellwand oder zellumgebende Strukturen wie Peptidoglukanen vom umgebenden Medium getrennt. Bei Bakterien, die nicht über einen Zellkern verfügen und deren gesamte Nukleinsäuren demzufolge im Cytoplasma vorliegen, sind die Nukleinsäuren durch Plasmamembran und Peptidoglycan-Zellwand sowie ggf. einer Lipopolysaccharidschicht vom umgebenden Medium getrennt. Sowohl bei eukaryontischen als auch bei prokaryontischen Zellen führt die Lyse in physiologischer Hinsicht zum Zelltod. Der Begriff "lysieren" bezeichnet somit jede Tätigkeit, die zur Lyse der Zellen oder Gewebe führt.

Das erfindungsgemäße Verfahren umfasst Schritte, bei denen man zunächst die höheren eukaryontischen Zellen und/oder Gewebe und in einem späteren Schritt auch die prokaryontischen oder eukaryontischen mikrobiellen Zellen in der Mischprobe lysiert. Die Nukleinsäuren werden dabei aus den jeweiligen Zellen bzw. Gewebe freigesetzt. Im Rahmen der vorliegenden Erfindung wird die Lyse der höheren eukaryontischen Zellen und/oder Gewebe (Schritt a des Verfahrens) derart durchgeführt, dass es nicht zu einer Lyse der in der Probe befindlichen mikrobiellen Zellen kommt. Diese bleiben im wesentlichen intakt, d.h. die äußere Begrenzung dieser Zellen (Zellwand oder ähnliche Strukturen) wird durch die in Schritt a) des erfindungsgemäßen Verfahrens angewendete Lyse-Methode nicht zerstört, so dass keine Nukleinsäuren aus den mikrobiellen Zellen freigesetzt werden.

Im ersten Schritt des erfindungsgemässen Verfahrens der gezielten Aufreinigung von Nukleinsäure aus mikrobiellen Zellen werden die in der Probe enthaltenen höheren eukaryontischen Zellen und/oder Gewebe lysiert.

Dabei werden alle oder zumindest ein erheblicher Teil der in der Probe befindlichen höheren eukaryontischen Zellen und/oder Gewebe lysiert. Es ist bevorzugt, dass im ersten Schritt des Verfahrens Bedingungen angelegt werden, die dazu führen, dass über 30% der in der Probe enthaltenen höheren eukaryontischen Zellen und/oder Gewebe lysiert werden, wobei es insbesondere bevorzugt ist, dass mehr als 40%, 50%, 60%, 70%, 80%, 90%, 95% oder 99% der in der Probe befindlichen höheren eukaryontischen Zellen und/oder Gewebe lysiert werden. Um den Anteil an höheren eukaryontischen Zellen und/oder Gewebe zu ermitteln, die unter den gewählten Bedingungen im ersten Schritt des erfindungsgemäßen Verfahrens lysiert werden, können Kontrollversuche durchgeführt werden, die quantitative Aussagen hinsichtlich der Lyse der Zellen erlauben. Das Ausmaß der Lyse der höheren eukaryontischen Zellen und/oder Gewebe unter den im ersten Schritt des erfindungsgemäßen Verfahrens gewählten Bedingungen kann beispielsweise unter Verwendung einer quantitativen PCR-Reaktion erfasst werden. Dazu führt man zunächst bei einem Aliquot einer definierten Probe den Lyse-Schritt durch und reinigt die freigesetzte Nukleinsäure mittels herkömmlicher Verfahren. Die gesamte gereinigte Nukleinsäure kann anschließend als Template in einer PCR oder RT-PCR für die Amplifikation eines Gens verwendet werden (z.B. Cytochrom B), das spezifisch ist für höhere Eukaryonten. Durch Vergleich mit den Amplifikationsprodukten aus einem weiteren Aliquot derselben Probe, die nach üblichen Verfahren vollständig aufgeschlossen wurde, lassen sich Aussagen hinsichtlich des Ausmaßes der Lyse unter den gewählten Bedingungen treffen.

Die im ersten Schritt des Verfahrens anzulegenden Bedingungen werden so gewählt, dass die in der Mischprobe enthaltenen mikrobiellen Zellen intakt bleiben. Dies bedeutet, dass die in der Probe befindlichen mikrobiellen Zellen nur in einem geringen Ausmaß unter den gewählten Bedingungen lysieren dürfen.

Solche selektiven Bedingungen können beispielsweise über die Konzentration von lysierenden Agenzien eingestellt werden (siehe nachfolgend). Es wird bevorzugt, dass unter den gewählten Bedingungen lediglich ein Anteil von 15% oder weniger der mikrobiellen Zellen in der Mischprobe lysiert wird. Gemäss einer besonders bevorzugten Ausführungsform beträgt der Anteil der lysierten mikrobiellen Zellen im ersten Schritt weniger als 40%, 30%, 20%, 10%, 5%, wobei weniger als 3% oder weniger als 1% besonders bevorzugt sind. Das Ausmaß der Lyse von bestimmten mikrobiellen Zellen bei definierten Lyse-Bedingungen lässt sich durch hinreichend bekannte Verfahren ermitteln, beispielsweise durch Verwendung einer Reinkultur des jeweiligen Organismus unter den ausgewählten Lyse-Bedingungen bei (gleichzeitiger oder anschließender) Messung der optischen Dichte bei einer Wellenlänge von 260 nm. Bei dieser Wellenlänge weisen Nukleinsäuren ein charakteristisches Absorptionsmaximum auf, sodass die Lyse der Zellen über die Freisetzung von Nukleinsäuren aus diesen Zellen quantifiziert werden kann. Alternativ können auch die oben im Zusammenhang mit den höheren eukaryontischen Zellen und/oder Geweben beschrieben PCR-Verfahren verwendet werden.

Verfahren zur Lyse sind im Stand der Technik hinreichend für verschiedene Zellarten und auch Gewebe beschrieben. So lassen sich bakterielle Zellen beispielsweise durch wiederholtes Auftauen und Einfrieren, Behandlung mit Enzymen wie Lysozym oder Lysostaphin, mechanische Behandlung in einer French-Presse oder Zellmühle, Ultraschall-Behandlung oder ähnliche Methoden lysieren. Bestimmte eukaryontische Zellen, wie z.B. Hefezellen lassen sich beispielsweise durch mechanische Behandlung mit Glaskügelchen oder enzymatische Behandlung mit Zymolase (Lyticase) lysieren. Pilzzellen lassen sich durch mechanische Behandlung wie Mörsern unter flüssigem Stickstoff oder enzymatische Behandlung mit Chitinase lysieren. Pflanzliche Gewebe werden im allgemeinen durch Mörsern unter flüssigem Stickstoff und enzymatische Behandlung mit Proteasen lysiert.

Verschiedene andere eukaryontische Zellen, wie beispielsweise tierische Zellen können oftmals durch Osmolyse in einem hypotonischen Puffer oder durch elektrische Homogenisatoren oder enzymatische Behandlung mit Proteasen lysiert werden. Annährend jede Art von Zelle oder Gewebe kann durch Zugabe bestimmter, aggressiver chemischer Agenzien (wie z.B. chaotroper Salze oder Detergenzien) lysiert werden.

Gemäß einer besonders bevorzugten Ausführungsform der vorliegenden Erfindung erfolgt die Lyse der höheren eukaryontischen-Zellen und/oder Geweben in der Mischprobe durch Zugabe eines lysierenden Agens. Bei dem lysierenden Agens kann es sich um eine chemische Substanz (oder eine Mischung von Substanzen) handeln, die die Membran- und/oder Zellwand-Struktur von Zellen bzw. Geweben zerstört. Die Bedingungen werden so gewählt, dass das lysierende Agens zunächst in einer Konzentration eingesetzt wird, die zur Lyse von höheren eukaryontischen Zellen und Geweben führt, so dass die Nukleinsäuren aus diesen freigesetzt werden. Die Konzentration des jeweiligen Agens wird so gewählt, dass die in der selben Probe enthaltenden mikrobiellen Zellen (beispielsweise Bakterien) nicht angegriffen werden.

Das lysierende Agens kann dabei beispielsweise in einer Lösung vorliegen, die zu der jeweiligen Mischprobe gegeben wird. Die Lösung kann ferner weitere Inhaltsstoffe umfassen, die lysierende Wirkung der jeweils verwendeten Agenzien verstärkt und/oder unterstützt. Vorzugsweise liegen die lysierenden Agenzien in einer gepufferten Lösung vor, die einen oder mehrere der folgenden Substanzen umfassen kann: Puffer wie beispielsweise TRIS (Tris(hydroxymethyl)-aminomethan), MOPS (3-(N-Morpholino)-Propansulfonsäure) oder HEPES (N-(2-Hydroxyethyl)-piperazin-N'-ethansulfonsäure), chelierende Agenzien wie EDTA (Ethylendiamintetraessigsäure), EGTA (Ethylenglycol-bis(β-Aminoethylether)-N,N,N',N'-Tetraessigsäure).

Gemäss einer besonders bevorzugten Ausführungsform handelt es sich bei dem lysierenden Agens um ein oder mehrere chaotrope Agenzien und/oder um ein oder mehrere Tenside, d.h. die Lyse der Zellen kann sowohl mit einem oder mehreren chaotropen Agenzien oder mit einem oder mehreren Tensiden durchgeführt werden. Bei den chaotropen Agenzien, die zur Verwendung bei dem ersten Lyse-Schritt in Betracht kommen, kann es sich um chaotrope Salze, wie beispielsweise Guanidin-Hydrochlorid, Natriumiodid, Guanidin-Isothiocyanat oder Mischungen derselben handeln. Eingesetzt werden können Guanidinsalze bis 4-5 mol/l und Tenside bis 20 Gew.%, ohne dass mikrobielle Zellen lysieren (<40%); bevorzugt werden 1 M Guanidinsalze und 1 Gew.% Tenside (in Hinsicht auf den Einsatz der Endonuklease).

Es wurde im Rahmen der vorliegenden Erfindung überraschenderweise herausgefunden, dass Mischproben, die humane Blutzellen .und humane Zellen von vaskulärem Gewebe einerseits und Bakterienzellen anderseits umfassen, durch Inkubation mit chaotropen Salzen, wie Guanidin-Hydrochlorid und Guanidin-Isothiocyanat, derart behandelt werden können, dass nur die humanen Zellen lysiert werden, nicht hingegen die bakteriellen Zellen. Gemäss einer besonders bevorzugten Ausführungsform der vorliegenden Erfindung handelt es sich somit bei dem lysierenden Agens um ein Gemisch aus dem chaotropen Salz Guanidin-Hydrochlorid und dem Tensid Tween-20. Gemäß einer besonders bevorzugten Ausführungsform handelt es sich bei dem lysierenden Agens um eine Mischung von Guanidin-Hydrochlorid in einer Endkonzentration von 1 mol/l und Tween-20 in einer Menge von 1 Gewichtsprozent.

In einem nachfolgenden Schritt werden die mikrobiellen Zellen, die unversehrt aus der Lyse in Schritt a) des erfindungsgemäßen Verfahrens hervorgegangen sind, von den lysierten höheren eukaryontischen Zellen getrennt. Zum Zwecke dieser Trennung können im Stand der Technik hinreichend bekannte Verfahren verwendet werden.

Gemäss einer Ausführungsform der Erfindung erfolgt die Abtrennung der mikrobiellen Zellen durch Zentrifugation. Dabei können übliche Rotationsgeschwindigkeiten verwendet werden, die in der Lage sind, bakterielle Zellen in einem entsprechenden Zentrifugengefäß zu sedimentieren, während die gelösten Bestandteile der lysierten eukaryontischen Zellen und/oder Gewebe im Überstand verbleiben. Solche Rotationsgeschwindigkeiten liegen in der Regel in einem Bereich von 1.000-15.000 x g.

Gemäss einer alternativen Ausführungsform erfolgt die Abtrennung der mikrobiellen Zellen von den lysierten höheren eukaryontischen Zellen und/oder Geweben durch Filtration. Filtrationsverfahren sind dem Fachmann hinlänglich bekannt und umfassen beispielsweise die Filtration durch Filter, die eine Porengrösse aufweisen, welche es erlaubt, kleinere Zellbestandteile, Zelltrümmer und Flüssigkeiten von intakten mikrobiellen Zellen wirkungsvoll zu trennen. Entsprechend geeignete Filter sind beispielsweise von der Firma Sartorius unter der Bezeichnung Sartobran mit Porengrössen von 0,2 - 0,45 µm erhältlich. Die Filtermaterialien können aus Gründen der einfachen Handhabung auch in Form üblicher Prep-Spin-Säulen vorliegen.

Nach Abtrennung der mikrobiellen Zellen von den lysierten höheren eukaryontischen Zellen und/oder Geweben wird die Lyse der mikrobiellen Zellen durchgeführt (Schritt c des erfindungsgemäßen Verfahrens). Die Lyse kann durch alle dem Fachmann bekannte Methoden durchgeführt werden, die zur Lyse der jeweiligen Zellen geeignet ist. Gemäß einer bevorzugten Ausführungsform der vorliegenden Erfindung erfolgt die Lyse der mikrobiellen Zellen durch mechanische Verfahren. Mechanische Verfahren zum Aufschluss von Zellen sind hinlänglich bekannt und umfassen u.a. das Homogenisieren mittels eines Mörsers bzw. mittels elektrischer oder mechanischer Apparaturen, wie beispielsweise durch Behandlung mit Ultraschall, French-Presse, Zellmühle.

Gemäß einer weiteren Ausführungsform der vorliegenden Erfindung erfolgt die Lyse der mikrobiellen Zellen in Schritt c) des erfindungsgemäßen Verfahrens enzymatisch. Hierbei kann in Abhängigkeit von dem jeweils nachzuweisenden Organismus ein Enzym gewählt werden, welches zum Auflösen der Zellwand- oder äußeren Begrenzungsstruktur führt. Dabei kann es sich beispielsweise bei Prokaryonten um Lysozym, bei Hefen um Lyticase, bei Pilzen um Chitinasen, bei Algen um Cellulasen und bei Protozoen um Proteasen handeln. Sollen beispielsweise Bakterien mit eigentümlicher Zellwandstruktur lysiert werden, kommen auch andere Enzyme zur Verwendung im Rahmen des erfindungsgemäßen Verfahrens in Betracht, z.B Lysostaphin zur Auflösung der Staphylococcen-Zellwand. Ferner können auch Proteasen bei der Lyse sowohl mikrobieller prokaryontischer wie auch mikrobieller eukaryontischer Zellen verwendet werden. Selbstverständlich kann die Lyse der mikrobiellen Zellen auch durch ein Verfahren erfolgen, das mechanische und enzymatische Behandlungen einschließt. Zur Lyse der mikrobiellen Zellen können auch die bereits oben erwähnten chaotropen Angenzien und/oder Tenside in Konzentrationen benutzt werden, die die oben definierten Konzentrationen der Agenzien bei der Lyse der höheren eukaryontischen Zellen übersteigen. So können beispielsweise Guanidinhydrochlorid oder Guanidinisothiocyanat in Konzentrationen von über 4 mol/l oder 5 mol/l im jeweiligen Ansatz verwendet werden; bei diesem Schritt können sogar noch höhere Konzentrationen verwendet werden, die bis zur Löslichkeitsgrenze der entsprechenden Substanz reichen. Den chaotropen Agenzien können ferner auch Tenside zugesetzt werden. Auch kann eine Kombination aus enzymatischer Behandlung und dem Einsatz von chaotropen Agenzien und/oder Tensiden auch innerhalb der für die Lyse von höheren eukaryontischen Zellen bzw. Geweben eingesetzten Konzentrationen angewendet werden, da durch die enzymatische Behandlung die Zellwände bzw. äußeren Zellbegrenzungsstrukturen entfernt werden und Zellmembranen sich durch chaotrope Salze und/oder Tenside auflösen, was zur Lyse der mikrobiellen Zellen führt.

Abschließend erfolgt die Reinigung der aus mikrobiellen Zellen freigesetzten Nukleinsäuren. Diese kann mit Hilfe im Stand der Technik bekannten Verfahren, wie beispielsweise Phenol-Chloroform-Extraktion, erfolgen. In den letzten Jahren sind zunehmend alternative Reinigungsverfahren für Nukleinsäuren entwickelt worden, die sich durch eine einfache Handhabung sowie geringere gesundheitliche Risiken auszeichnen (z.B. durch Verzicht auf Lösungsmittel wie Chloroform oder Phenol). Dabei werden die Nukleinsäuren üblicherweise in Gegenwart hoher Ionenstärke (z.B. in Gegenwart von chaotropen Salzen) an mineralische Trägermaterialien, wie beispielsweise Glas-Partikel, fein gemahlenes Glaspulver, Diatomeenerde oder Silikagele gebunden, gewaschen und schließlich in einem geeigneten Puffer eluiert. Diese Art von Nukleinsäure-Reinigungssytem ist in Form von Kits kommerziell erhältlich, z.B. von der Molzym GmbH & Co KG (Bremen, DE), Qiagen (Hilden, DE), Macherey-Nagel (Düren, DE), Roche (Basel, CH) oder Sigma (Deisenhofen, DE). Nukleinsäure-Reinigungssysteme, die chaotrope Salze verwenden, lassen sich besonders vorteilhaft im Rahmen des erfindungsgemäßen Verfahrens verwenden, da nach den Lyse-Schritten a) und c) die freigesetzten, mikrobiellen Nukleinsäuren bereits in einem hochmolaren chaotropen Milieu vorliegen. Allerdings können auch andere Systeme zur Nukleinsäure-Reinigung, die auf der Verwendung von Polystyrol-Kugeln, Nitrocellulose-Papier u.ä. als Trägermaterial basieren, im Rahmen der vorliegenden Erfindung verwendet werden.

Das Verfahren der vorliegenden Erfindung ermöglicht die Bereitstellung von Nukleinsäuren von mikrobiellen Zellen aus Mischproben in einem Reinheitsgrad, der einen höchst möglichen Grad an Störungsfreiheit bei nachfolgenden Verfahren, insbesondere bei diagnostischen Verfahren zum Nachweis bestimmter pathogener Bakterien, gewährleistet. Es ist bevorzugt, dass es sich bei der Nukleinsäure, die aus den mikrobiellen Zellen isoliert wird, um DNA und/oder RNA handelt.

Gemäss dem Verfahren der vorliegenden Erfindung kann die untersuchte Mischprobe Körperflüssigkeiten wie beispielsweise Blut, Urin, Stuhl, Sputum, Lavage, Punktat, Wundabstrich, Lymphe und/oder Sekret menschlichen oder tierischen Ursprungs und/oder Gewebe menschlichen oder tierischen Ursprungs oder Teile davon umfassen. Gemäß einer besonders bevorzugten Ausführungsform handelt es sich bei den höheren eukaryontischen Zellen, die in der Mischprobe vorliegen, um Blutzellen oder Gewebezellen menschlichen oder tierischen Ursprungs. Gemäß einer besonderen Ausführungsform kann es sich bei der Mischprobe auch um Blutprodukte, insbesondere Thrombozytenkonzentrate und Erythrozytenkonzentrate, oder um andere, im Rahmen der Infektionsdiagnostik isolierte Körperflüssigkeiten, insbesondere Punktate (z.B. Gelenk, Augen), Liquor und Bronchial-Alveolar-Lavage, handeln. Ferner kann die Mischprobe Gewebe pflanzlichen Ursprungs oder Teile davon umfassen. Die Probe kann Gewebe von mit Bakterien, Hefen, Pilzen oder Protozoen infizierten Patienten, mit pathogenen Bakterien oder Pilzen infizierten Pflanzen oder Tieren umfassen. Gemäß einer besonders bevorzugten Ausführungsform enthält die Mischprobe Blut eines mit einem Bakterium infizierten Menschen oder Tiers. Es gibt darüber hinaus auch symbiontische Systeme, zum Beispiel bei Schwämmen, die mit Bakterien eine enge Lebensgemeinschaft bilden und Ansatz für analytische und biotechnologische Fragestellungen sind.

Das erfindungsgemäße Verfahren ist daneben auch auf den Nachweis von mikrobiellen Zellen bzw. Organismen in Pflanzenmaterial geeignet. Somit lassen sich insbesondere pflanzenpathogene oder -besiedelnde prokaryontische Organismen, wie beispielsweise bestimmte Bakterienarten der Gattung Mycoplasma, Clavibacter, Xanthomonas, Corynebacterium, Acidovorax, Brenneria, Burkholderia, Rhizobium oder Agrobacterium u.a., in Mischroben mit pflanzlichem Material unmittelbar nachweisen. Gemäß einer besonderen Ausführungsform umfasst die Mischprobe Pflanzenmaterial, insbesondere Material von Blatt, Stängel, Wurzel, Samen und Früchten.

Gemäß einer weiteren bevorzugten Ausführungsform wird die freigesetzte Nukleinsäure aus den höheren eukaryontischen Zellen im Anschluss an die Lyse der eukaryontischen Zellen in Schritt a) aus der Probe entfernt.

Dies kann beispielsweise unter Verwendung herkömmlicher Filtrationsmethoden erfolgen, wie sie im Stand der Technik hinreichend bekannt sind. Beispielsweise kann die Nukleinsäure mit Hilfe üblicher Kits zur Nukleinsäure-Aufreinigung aus der Probe entfernt werden. Dabei können dieselben Kits verwendet werden, die anschließend zur Reinigung der Nukleinsäuren aus den prokaryontischen Zellen gewonnen wird. Somit betrifft die vorliegende Erfindung gemäß eines besonderen Aspekts auch ein Verfahren zur sequentiellen Isolierung von Nukleinsäure aus höheren eukaryontischen und mikrobiellen Zellen, bei dem zunächst die Nukleinsäuren aus höheren eukaryontischen Zellen freigesetzt und gereinigt werden und in einem nachfolgenden Schritt die Nukleinsäuren aus mikrobiellen Zellen freigesetzt und gereinigt werden.

Sofern für die höheren eukaryontischen Nukleinsäuren keine weitere Verwendung besteht, kann diese auch aus der Probe entfernt werden, indem sie abgebaut wird. Dies ist insbesondere bei nachfolgenden Applikationen der jeweiligen Ziel-Nukleinsäure von Vorteil, die einen hohen Reinheitsgrad der Ziel-Nukleinsäure und folglich eine möglichst quantitative Entfernung anderer Nukleinsäure erfordern (z.B. PCR). Der Abbau findet dabei vorzugsweise durch enzymatische Behandlung der Probe mit einer wie oben beschriebenen DNA-abbauenden Nuklease statt. Die Nuklease kann bereits zu Beginn der Lyse der höheren eukaryontischen Zellen zugesetzt werden (z:B. zeitgleich mit der Zugabe der lysierenden Substanzen zu der Probe) oder kann nach erfolgter Lyse (d.h. nach einer Inkubationsphase) zugegeben werden.

Die vorliegende Erfindung stellt ferner die Verwendung eines Kits zur Durchführung eines Verfahrens zur Isolierung und/oder Aufreinigung von RNA aus Mischungen bereit, wobei der Kit eine DNA-abbauenden Nuklease umfasst, die keine RNAse-Aktivität aufweist und in der Lage ist, in Gegenwart von einem oder mehreren chaotropen Agenzien und einem oder mehreren Tensiden DNA abzubauen, sowie Puffer und Reagenzien für die Isolierung und/oder Aufreinigung von RNA umfasst, und wobei die Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

Gemäß einer bevorzugten Ausführungsform handelt es sich bei der Nuklease um das in SEQ ID NO:1, SEQ ID NO:2, SEQ ID NO:3 oder SEQ ID NO:4 gezeigte Polypeptid. Die Erfindung betrifft ferner die Verwendung eines Kits, der eine wiechen definierte DNA-abbauende Nuklease umfasst, die in der Lage ist, in Gegenwart von einem oder mehreren chaotropen Agenzien und/oder einem oder mehreren Tensiden DNA abzubauen, zur Isolierung und/oder Aufreinigung von RNA.

Die vorliegende Erfindung stellt ferner die Verwendung eines Kits zur Durchführung eines Verfahrens zur Isolierung von Nukleinsäure aus mikrobiellen Zellen bereit, die in einer Mischprobe vorliegen, die höhere eukaryontische Zellen und/oder Gewebe umfasst. Der Kit umfasst eine DNA-abbauende Nuklease, die keine RNAse-Aktivität aufweist und in der Lage ist, in Gegenwart von einem oder mehreren chaotropen Agenzien und einem oder mehreren Tensiden DNA abzubauen, und Puffer und Reagenzien für die Isolierung und/oder Aufreinigung von RNA umfasst, wobei die Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

Die Erfindung betrifft ferner die Verwendung eines solchen Kits zur Durchführung eines Verfahrens zur Isolierung von Nukleinsäure aus mikrobiellen Zellen bereit, die in einer Mischprobe vorliegen, die höhere eukaryontische Zellen und/oder Gewebe umfasst.

Die Erfindung wird nachfolgend anhand von Beispielen beschrieben, die der Illustration dienen, ohne die Erfindung darauf zu beschränken. Für den Fachmann wird ersichtlich sein, dass Modifikationen und Variationen der beschriebenen Beispiele möglich sind, ohne vom Erfindungsgedanken abzuweichen.

### BEISPIELE

### A. Charakteristika der Endonuklease I aus E. coli

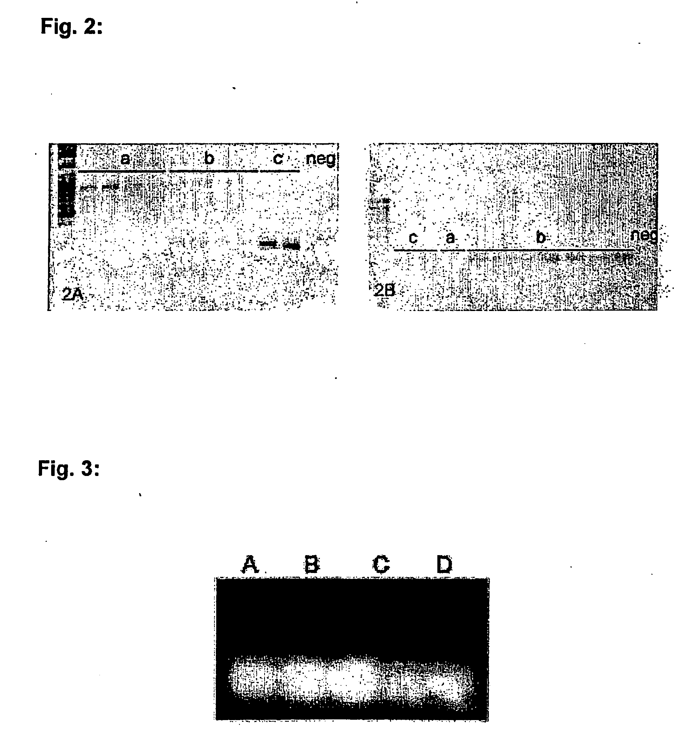

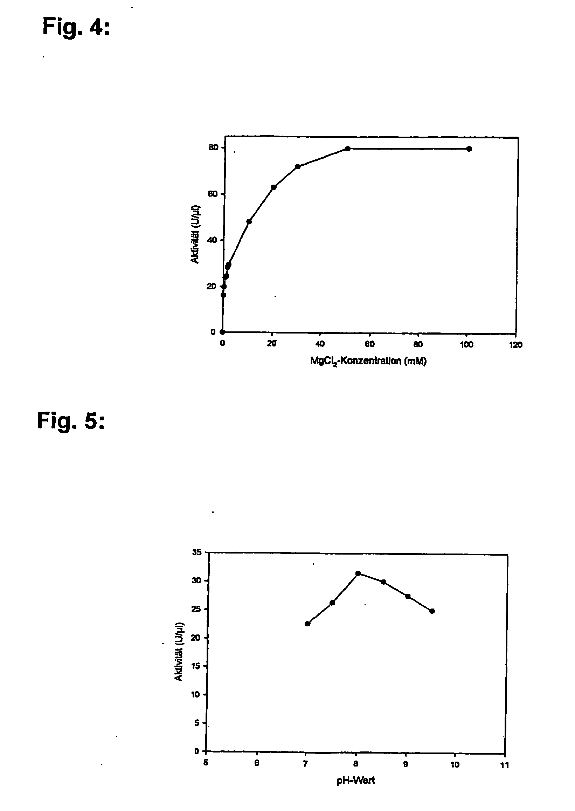

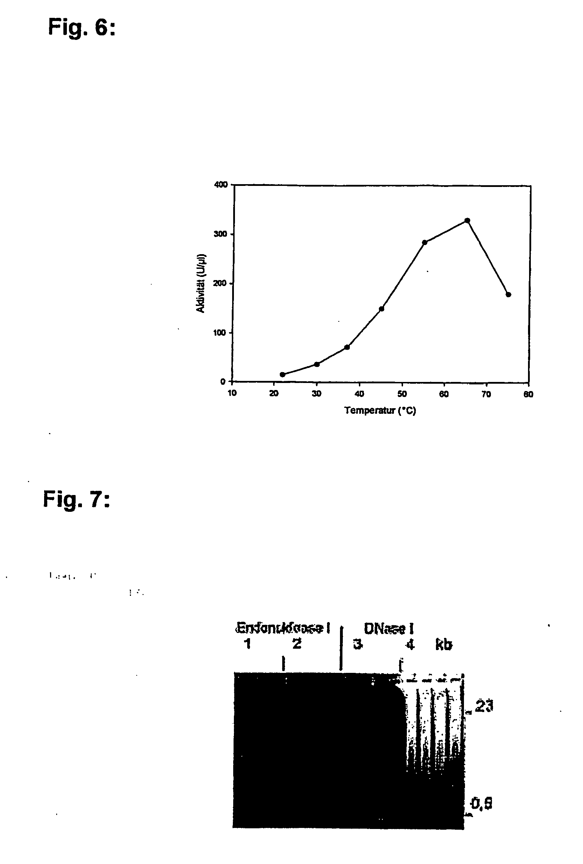

Es wurden die allgemeinen biochemischen Charakteristika der Endonuklease I aus E. coli (SEQ ID NO: 1) untersucht. Das Enzym besitzt chaotrop-resistente Eigenschaften (Guanidin-Hydrochlorid bzw. -Isothiocyanat). Es weist eine Endonuklease-Aktivität ohne RNase-Aktivität auf (siehe Fig.3). Als Kofaktor werden Magnesium-Ionen benötigt bei einem Optimum von 50 mmol/l (siehe Fig. 4). Das pH-Wert-Optimum des Enzyms liegt bei pH 8,0 (siehe Fig. 5). Das Temperaturoptimum des Enzyms beträgt 65 °C (siehe Fig. 6). Die Endonuklease ist im Vergleich zu gängigen, kommerziell verfügbaren Endonukleasen (z.B. DNase I, Firma Roche, Basel, CH und Benzonase, Merck, Darmstadt) deutlich resistenter gegen chaotrope Bedingungen wie 1 mol/l Guanidin-Hydrochlorid bzw. 1 mol/l Guanidin-Isothiocyanat (siehe Fig. 7).

### B. Nukleinsäure-Reinigung

Für die nachfolgend dargestellten Nukleinsäure-Reinigungsversuche wurden folgende Materialien verwendet:
Für die nachfolgend dargestellten Nukleinsäure-Isolierungsversuche wurden folgende Materialien verwendet:
   - Puffer 1 (5 mol/l Guanidinhydrochlorid, 5 % Tween-20, 50 mmol/l Tris-HCl, pH 8,0);
   - Puffer 2 (0,3 mol/l MgCl₂, 50 mmol/l Tris-HCl, pH 8);
   - Puffer 3 (5 mol/l Guanidinisothiocyanat, 5 % Tween-20 (v/v), 50 mmol/l Tris-HCl, pH 8);
   - Puffer 4 (50 mmol/l Tris-Cl, pH 8, 10 mmol/l EDTA, pH 8,0);
   - Puffer 5 (50 mmol/l Tris-Cl, pH 8,0, 10 mmol/l EDTA, pH 8,0, 20% (w/v) Saccharose);
   - Puffer 6 (50 mmol/l Tris-Cl, pH 8,0, 10 mmol/l EDTA, pH 8,0, 6,2 mmol/l CaCl₂, 1,25% (w/v) Natrium-Dodecylsulfat) Neu
   - Lysozym (100 mg/ml; >100.000 U/mg; erhältlich von der Molzym GmbH & Co KG, Bremen, Deutschland);
   - Endonuklease I von Escherichia coli; SEQ ID NO:1 (500 U/µl; erhältlich von der Molzym GmbH & Co KG, Bremen, Deutschland).

### Arbeitsvorschrift:

1. 10 ml frisches Vollblut (mit EDTA, Heparin oder Citrat stabilisiert) oder Konzentrate von prozessiertem Blut, einschließlich Thrombozyten- oder Erythrocytenanreicherungen, die mit Bakterien oder natürlichen Proben versetzt waren, wurden in ein steriles 50 ml Falcon-Röhrchen pipettiert, und es wurden 3,5 ml Puffer 1 zugegeben. Man mischte mittels Vortexer bei voller Geschwindigkeit für 5 Sekunden und ließ für 5 bis 10 Minuten stehen.
2. Dem Lysat fügte man 3,5 ml Puffer 2 und 10 µl *Endonuclease* I (500 U/µl) zu, wobei man den Puffer und das Enzym optional vor Verwendung kurz vormischte. Anschließend wurde sofort für 5 Sekunden gemischt (Vortexer). Man ließ für 15 Minuten stehen, wobei die Temperatur 20 bis 25 °C betrug.
3. Die Bakterienzellen wurden durch Zentrifugieren für 10 Minuten in einer Hochgeschwindigkeitszentrifuge bei 11.000 x g geerntet. Danach wurde der Überstand durch Dekantieren entfernt.
4. Im nächsten Schritt gab man 1 ml Puffer 4 zu, mischte (Vortexer) bis sich das Sediment resuspendierte und überführte die Suspension in ein steriles 1,5 oder 2 ml Polypropylen-Röhrchen. Durch Zentrifugieren bei Höchstgeschwindigkeit (>13.000 rpm) für 10 Minuten brachte man die Bakterienzellen zum Sedimentieren. Der Überstand wurde durch vorsichtiges Abpipettieren entfernt. Es zeigte sich, dass das Sediment in vielen Fällen kaum sichtbar war. Man mischte die Probe kräftig (Vortexer) für 10 bis 20 Sekunden. In manchen Fällen kam es vor, dass das Pellet aus Blutzellfragmenten und Bakterienzellen bestand, was ein aufwändiges und schwieriges Resuspendieren zur Folge hatte. Man rührte in diesem Fall mit einer Pipettenspitze und pipettierte mehrfach, bis man eine homogene Suspension erhalten hatte. Dieses Waschen diente dem Entfernen von Resten des chaotropen Agens und ermöglicht die enzymatische Behandlung als Teil der Nukleinsäureextraktion aus dem Pellet (siehe unten).
5. Man resuspendierte das Pellet in 90 µl Puffer 5 durch mehrfaches Pipettieren. Dabei konnten kleine Partikel in der Suspension vernachlässigt werden, da sie sich während der enzymatischen Behandlungen, insbesondere beim Proteinase K-Verdau, auflösten (siehe unten).
6. Anschließlich erwärmte man das Röhrchen für 5 Minuten auf 80°C, um *Endonuclease I* zu inaktivieren, und ließ dann auf Raumtemperatur abkühlen.
7. Man gab 10 µl Lysozym (oder andere Enzyme für den Zellwandabbau wie Mutanolysin oder Lysostaphin) zu und inkubierte das Röhrchen für 45 min bei 45°C.
8. Es erfolgte die Zugabe vom 10 µl Proteinase K und 150 µl Puffer 6. Es wurde mittels eines Vortexers bei Höchstgeschwindigkeit für 5 Sekunden gemischt und für 30 Minuten bei 45°C inkubiert.
9. Es wurden 250 µl Puffer 3 zugegeben und mittels eines Vortexers bei Höchstgeschwindigkeit für 5 Sekunden gemischt, danach für 5 Minuten bei Raumtemperatur stehen gelassen. Die Zellen lysierten, und das Protein denaturierte.

Die Aufreinigung der DNA erfolgte mit einem kommerziellen Kit zur DNA-Aufreinigung (PrestoSpin D Bug Kit, Molzym, Bremen, Deutschland) gemäß den Anweisungen des Herstellers.

### 1. Probenvorbereitung:

Folgende Proben wurden für die nachfolgenden Isolierungsverfahren verwendet. Ein Aliquot von 200 µl einer Vollblut-Probe humanen Ursprungs wurden in einem 1,5 ml Kunststoff- Zentrigugationsröhrchen mit 1 µl einer Bakteriensuspension (ca. 1x10⁹ bis zu 4x10⁹ Zellen/ml) versetzt und 5 sec mittels eines Vortexers gemischt ("spiked Vollblut"). Zu Vergleichszwecken wurde ferner ein weiteres 200 µl-Aliquot der selben Vollblut-Probe ohne Bakterien ("unspiked Vollblut") verwendet. Daneben wurde durch Zugabe von 1 µl einer Zellsuspension mit einer Konzentration von ca. 1x10⁹ bis zu 4x10⁹ Zellen pro ml zu 200 pl Puffer 4 eine weitere Vergleichsprobe hergestellt ("Reinkultur"). Desweiteren wurden 200 µl Proben von septischen Blutproben, Punktaten (= aspirates; z.B. Gelenk, Augen, Eiter) und Blutprodukten wie Thrombozyten- und ErythrozytenKonzentraten, gespikt oder natürlich, für das folgende Protokoll verwendet.

### 2. Isolierungsverfahren

Den Proben ("spiked Vollblut"; "unspiked Vollblut" und "Reinkultur") wurde jeweils 50 µl Puffer 1 zugefügt, und die Proben wurden für 5 sec mittels eines Vortexers gemischt. Um eine optimale Lyse von Blutzellen zu erreichen, wurden die Proben 5 min stehen gelassen.

Anschließend wurden 50 µl Puffer 2 sowie 1 µl Endonuklease I zugegeben, 5 sec mittels eines Vortexers gemischt und 15 min bei Raumtemperatur (20-25 °C) inkubiert. Auf diese Weise wurde die humane DNA degradiert.

Es wurde eine Zentrifugation bei >13.000 rpm durchgeführt, der Überstand der Proben wurde dekantiert und Reste der Flüssigkeit wurden abpipettiert und verworfen. Anschließend wurden 400 µl Puffer 4 hinzugefügt, 10 sec mittels eines Vortexers gemischt und 5 bis 10 min bei 13.000 rpm zentrifugiert, um die Zellen zu sedimentieren. Der Überstand wurde dekantiert, und die Reste der Flüssigkeit wurden abpipettiert und verworfen.

Nachdem den Pellets 50 µl Puffer 4 und 5 µl Lysozymlösung zugegeben wurde, wurden diese durch Verrühren mit der Pipettenspitze und durch Aufsaugen und Ausstoßen vollständig resuspendiert. Anschließend wurden die Zellsuspensionen 15 min bei 37 °C inkubiert. Danach wurden 250 µl Puffer 3 zugegeben und nach 5 sec Mischen (Vortexer) wurden die Proben 5 min stehen gelassen, um eine optimale Lyse der Bakterienzellen und eine Inaktivierung von Resten der Endonuklease I zu bewirken.

### 3. Aufreinigung der Bakterien-DNA:

Die Aufreinigung der DNA erfolgte mit einem kommerziellen Kit zur DNA-Aufreinigung (PrestoSpin D Bug Kit, Molzym, Bremen, Deutschland) gemäß den Anweisungen des Herstellers.

Zu diesem Zweck wurden zunächst 200 µl Puffer AB zu den Proben gegeben und 5 sec gemischt (Vortexer). Anschließend wurde der Inhalt der jeweiligen Probenröhrchen in jeweils eine Spin-Säule dekantiert und 30 sec bei >13.000 rpm zentrifugiert. Der Durchlauf wurde jeweils verworfen. Die Säulen wurden durch Zugabe von 400 µl Puffer WB gewaschen und anschließend 30 sec bei >13.000 rpm zentrifugiert. Der Durchlauf wurde erneut verworfen.

Die Säulen wurden mit 400 µl 70% Ethanol gewaschen und anschließend getrocknet (3 min, >13.000 rpm).

Danach wurden die Spin-Säulen vorsichtig in sterilisierte (autoklaviert und getrocknet) 1.5 ml-Kunstoff-Zentrifugationsröhrchen eingebracht. 100 µl Puffer EB (70 °C) wurden jeweils auf die Filteroberfläche pipettiert und der Deckel wurde geschlossen. Nach einer einminütigen Inkubation wurde die DNA-Lösung durch Zentrifugation bei >13.000 rpm eluiert.

Die so aufgereinigte DNA wurde anschließend mittels Gelelektrophorese analysiert. Um die Vorzüge des erfindungsgemäßen Verfahrens zu verdeutlichen, wurde zu Vergleichszwecken jeweils ein Aliquot der unter 1. beschriebenen Proben ("spiked Vollblut", "unspiked Vollblut" und "Reinkultur") mit Hilfe eines kommerziellen Kits (DNeasy, Qiagen, Hilden, DE) nach dem herkömmlichen Prinzip der Gesamtextraktion behandelt.

### 4. Gelelektrophorese:

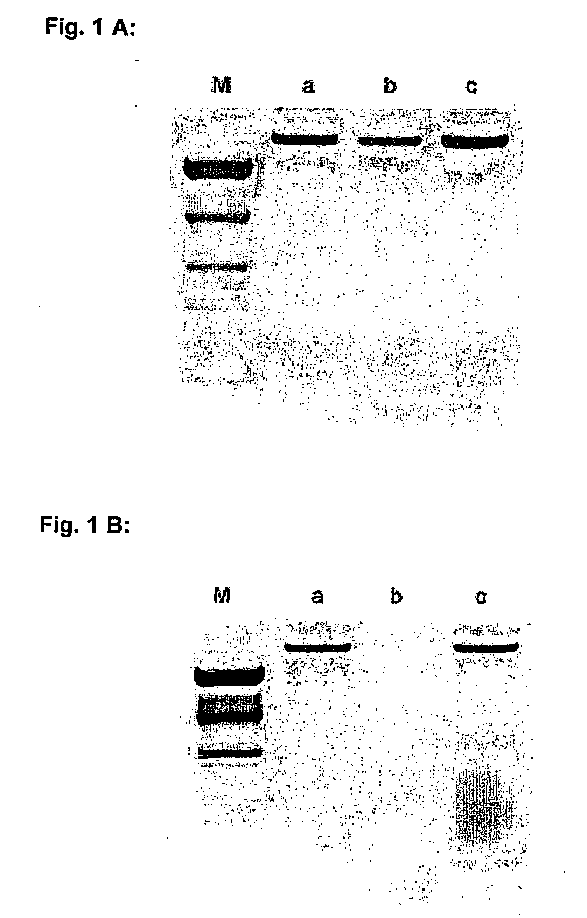

Die Ergebnisse der gelelektrophoretischen Trennung sind in den Figuren 1A und 1B dargestellt. Figur 1A zeigt das Ergebnis der gelelektrophoretischen Trennung der Nukleinsäuren aus Proben, die mittels des kommerziellen Kits nach dem Prinzip der Gesamtextraktion isoliert wurden. Es lässt sich erkennen, dass diese Methode zur Lyse der Bakterienzellen wie auch der Blutzellen führt, wodurch die DNA aus beiden Zelltypen freigesetzt wird.

Figur 1B zeigt das Ergebnis der elektrophoretischen Trennung der Nukleinsäuren aus Proben, die mittels des erfindungsgemässen Verfahrens behandelt wurden. Nach Lyse der Blutzellen erfolgte die Zugabe von Endonuklease I zum Abbau von DNA. Es lässt sich erkennen, dass die DNA der Blutzellen vollständig abgebaut wird. Die zu einem späteren Zeitpunkt von lysierten Bakterien freigesetzte hochmolekulare DNA wird aus der Mischprobe in Mengen und der Güte (hochmolekular) erhalten, die den

Mengen und der Güte bei der Kontolle entsprechen (vergleiche Bahnen der Proben "spiked Vollblut" und "Reinkultur").

### 5. PCR-Amplifikation

Die mit Hilfe des erfindungsgemässen Verfahrens bzw. mittels herkömmlicher Kits isolierten Nukleinsäuren, die jeweils ausgehend von einer Mischprobe aus Bakterien und Blut ("spiked Vollblut") aufgereinigt wurde, wurden im Folgenden für eine PCR unter den nachfolgend aufgeführten Bedingungen verwendet.

Die PCR wurde nach gängigen Verfahren zur Amplifikation von 16S rDNA-Sequenzen durchgeführt (25 µl PCR-Ansatz; Zyklen: 5 Minuten denaturieren bei 95°C, gefolgt von 30 Zyklen von 1 Minute Denaturieren bei 95°C, 1 Minute Annealing bei 55°C und 1 Minute Extension bei 72°C). Als Primer wurden z.B. 341f (SEQ ID NO: 5; 5'-CCT ACG GGA GGC AGC AG-3') und 985r (SEQ ID NO: 6; 5'-GTA AGG TTC TTC GCG TT-3') verwendet.

Figur 2A hingegen zeigt das Ergebnis der PCR unter Verwendung von Nukleinsäuren, die mit Hilfe des herkömmlichen Kits erhalten worden waren. Deutlich zu erkennen sind die unspezifischen Produkte, die im Zuge der Amplifikation in Proben entstanden sind, die humane Blutzellen enthielten ("unspiked" und "Spike").

Figur 2B zeigt das Ergebnis der PCR unter Verwendung von Nukleinsäuren, die mit Hilfe des erfindungsgemäßen Verfahrens isoliert worden waren. Es läßt sich deutlich erkennen, dass lediglich eine spezifische Bande für das 16S-rRNA-Produkt in den Proben zu erkennen ist, die Bakterien enthielten (nämlich in der Probe "spike": entspricht "spiked Vollblut"; und in der Probe "Reinkultur". Das Vollblut ohne Bakterienzusatz wies diese Bande nicht auf.

Dies verdeutlicht, dass mit Hilfe des erfindungsgemäßen Verfahrens die Blutzellen selektiv aufgelöst wurden und ihre Nukleinsäuren freigesetzt und von der Nuklease abgebaut wurden. Die bakteriellen Zellen dagegen bleiben während dieser Behandlung intakt und werden erst im nächsten Schritt aufgelöst, wodurch auch ihre Nukleinsäuren freigesetzt werden. Im vorliegenden Beispiel ist die Konzentration des die Guanidinisothiocyanat-Konzentration in Puffer 3 ausreichend hoch, um eine Inaktivierung der Endonuklease I zu bewirken.

### C. Isolierung von DNase-produzierenden Mikroorganismen aus extremen Habitaten

Als Verfahren zur Isolierung Nuklease-produzierender Mikroorganismen (Bakterien und Archaeen), die im Rahmen der vorliegenden Erfindung geeignet sind, wurden Wasser- und Sedimentproben aus salzhaltigen (>1 % NaCl bis 34% [Sättigung]) und alkalischen (pH > 7 bis 10) Standorten, z.B. eingetrocknete Seewasserpfützen oder Meerwasser, gesammelt. Die Proben wurden nach bekannten mikrobiologischen Verfahren auf agarhaltigen Medien ausgestrichen (z.B. Aufschütteln der Sedimentproben oder direkt Meerwasser und Auftragen auf Agarmedium, siehe unten). Die Zusammensetzung der Medien entsprach publizierten Rezepten (z.B. Can. J. Microbiol. 6: 165, 1960) oder wurde nach den vor Ort vorherrschenden, in der physiko-chemischen Analyse ermittelten Ionenzusammensetzungen und pH-Werten entworfen. Als besondere Ausführung mit gutem Erfolg eingesetzt kam ein Basismedium (SML) mit folgender Zusammensetzung zum Einsatz: 7,5 g/l Casaminoacids (Difco, Michigan), 10 g/l Hefeextrakt (Difco, Michigan), 11,6 g/l tri-Natrium-Citrat (Merck, Darmstadt), 0,13 mM Magnesiumsulfat (Merck, Darmstadt), 0,05 mM Calciumchlorid (Merck, Darmstadt), 28 mM Kaliumchlorid (Merck, Darmstadt), 0,18 mM Eisen-II-chlorid (Merck, Darmstadt) und 10 mM di-Natriumhydrogenphosphat (Merck, Darmstadt). Zur Verfestigung wurde dem Basismedium 15 g/l Agar (Difco, Michigan) beigefügt. Der pH-Wert, entsprechend der vor Ort gemessenen Daten, wurde mit 2 M NaOH auf Werte zwischen pH 8,0 und 9,5 eingestellt bzw. mit 75 mM Glycin (Merck, Darmstadt) gepuffert (pH 9,5). Das Medium wurde daraufhin nach üblichem Verfahren autoklaviert (20-30 min, 120 °C, 1 bar Überdruck). Entsprechend der am Beprobungsstandort gemessenen Salinitäten wurde dem Medium handelsübliches Speisesalz, das 3 Stunden bei 180 °C trocken sterilisiert wurde, in Konzentrationen von 2,9 bis 30 % zugesetzt. Vor dem Gießen in Petrischalen wurde dem Agar-Basismedium das Speisesalz zugesetzt und aufgelöst (SML-Agar).

Reinkulturen (Stämme) wurden erhalten, indem die nach wenigen Tagen bis zu zwei Wochen nach dem Plattieren von Probenmaterial auftretenden Kolonien nach bekannten mikrobiologischen Verfahren durch mehrere wiederholte Ausstriche auf Medien nach obiger Zusammensetzung vereinzelt wurden.

Zum Nachweis von extrazellulär gebildeten Nukleasen (RNasen und/oder DNasen) wurden die isolierten Stämme auf SML-Agar und angepassten Salzkonzentrationen ausgestrichen, das zusätzlich
a) zum Nachweis von DNasen Lachssperma DNA (0,5 g/l; ICN Biochemicals) und 50 mg/ml Methylgrün (ICN Biochemicals) oder
b) zum Nachweis von RNasen Typ VI Torula Hefe-RNA (ICN Biochemicals) enthielt.

Entsprechende Enzymaktivitäten wurden durch entfärbte Höfe um die Kolonien (DNasen) bzw. klare Höfe nach Überschichtung der Agarplatte mit 10 %-iger Salzsäurelösung (RNasen) sichtbar gemacht.

Auf die oben beschriebene Weise konnten über 1000 Isolate aus den Salinen gewonnen werden, unter ihnen auch solche, die Nuklease-Aktivität aufwiesen (Tabelle 1).

**Tabelle 1: Eigenschaften von ausgewählten Isolaten aus Salinen**

| Stammbezeichnung | Charakter | | | | | |
|---|---|---|---|---|---|---|
| | Gattung (16S rRNA) | Isolationsbedingungen | Wuchsbereich | Wuchsoptimum | DNase | RNase |
| | | % NaCl/pH | % NaCl | % NaCl | | |
| SJ5Ü/4 | Salinivibrio sp. | 12/8 | 2,9-12 | 2,9-12 | + | + |
| SJ5S/6 | unbekannt | 12/8 | 0,5-20 | 12 | + | + |
| EG2S/2 | Halobacillus sp. | 12/8 | 0,5-20 | 2,9 | + | + |
| SJ1/4 | unbekannt | 12/8 | 12-20 | 20 | + | - |
| SJ6/2 | Halomonas sp. | 12/8 | 2,9-12 | 2,9 | + | - |
| EG2S/11 | Deleya sp. | 12/9,5 | 2,9-12 | 12 | + | - |
| EG2S/14 | Deleya sp. | 12/9,5 | 2,9-12 | 12 | + | - |
| SJ6S/18 | Salinivibrio sp. | 12/9,5 | 2,9-12 | 2,9-12 | + | + |
| SJ6Ü/21 | unbekannt | 12/8 | 2,9-12 | 12 | + | + |

Die Tabelle 1 zeigt, dass ein breites Spektrum an Arten isoliert werden konnte, die Nukleasen bilden. Die Stämme besitzen entweder DNase- und RNase-Aktivität oder nur jeweils eine der beiden Aktivitäten (in Tabelle 1 nur DNase, andere Stämme besitzen nur RNase-Aktivität, nicht gezeigt). Diese Nukleasen waren z.T. bei sehr hohen Salzkonzentrationen, also unter chaotropen Bedingungen aktiv (z.B. SJ1/4 nur mit DNase-Aktivität bei 12-20% NaCl).

Die DNasen der Stämme SJ1/4 (unbekannte, neue Art; Tabelle 1) und EG2S/2 (Halobacillus sp., Tabelle 1) sollen hier stellvertretend für die Nukleasen der anderen Stämme in Bezug zur Resistenz gegenüber chaotropen Bedingungen beschrieben werden.

## Patentansprüche

1. Verwendung einer DNA-abbauenden Nuklease, die keine RNAse-Aktivität aufweist, zum Abbau von DNA in Gegenwart von einem oder mehreren chaotropen Agenzien und einem oder mehreren Tensiden, **dadurch gekennzeichnet, dass** die DNA-abbauende Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

2. Verwendung nach Anspruch 1, **dadurch gekennzeichnet, dass** die DNA-abbauende Nuklease
a) die in SEQ ID NO:1 gezeigte Aminosäuresequenz aufweist;
b) eine Aminosäuresequenz aufweist, bei der bis zu 40% der Aminosäuren von der in SEQ ID NO:1 gezeigten Aminosäuresequenz abweichen; oder
c) ein enzymatisch aktives Fragment von (a) oder (b) ist.

3. Verwendung nach den Ansprüchen 1 bis 2, **dadurch gekennzeichnet, dass** die chaotropen Agenzien chaotrope Salze sind.

4. Verwendung nach Anspruch 3, **dadurch gekennzeichnet, dass** die chaotropen Salze Guanidinhydrochlorid, Guanidinisothiocyanat und/oder Natriumiodid sind.

5. Verwendung nach den Ansprüchen 1 bis 4, **dadurch gekennzeichnet, dass** die Gesamtkonzentration der chaotropen Agenzien 0,1 mol/l bis 2,5 mol/l beträgt.

6. Verwendung nach den Ansprüchen 1 bis 5, **dadurch gekennzeichnet, dass** die Tenside Natriumdodecylsulfat, Brji40, Triton X-100 und/oder Tween20 sind.

7. Verwendung nach den Ansprüchen 1 bis 6, **dadurch gekennzeichnet, dass** die Gesamtmenge an Tensiden im Ansatz 0,1 - 2 Gew.-% beträgt.

8. Verfahren zur Isolierung und/oder Aufreinigung von RNA aus Mischungen, die RNA und DNA umfassen, bei dem man
a) die DNA unter Verwendung einer DNA-abbauenden Nuklease, die keine RNAse-Aktivität aufweist, in Gegenwart von einem oder mehreren chaotropen Agenzien und einem oder mehreren Tensiden abbaut; und
b) die RNA mittels geeigneter Verfahren isoliert bzw. aufreinigt,
**dadurch gekennzeichnet, dass** die DNA-abbauende Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

9. Verfahren nach Anspruch 8, **dadurch gekennzeichnet, dass** die DNA-abbauende Nuklease
a) die in SEQ ID NO:1 gezeigte Aminosäuresequenz aufweist;
b) eine Aminosäuresequenz aufweist, bei der bis zu 40% der Aminosäuren von der in SEQ ID NO:1 gezeigten Aminosäuresequenz abweichen; oder
c) ein enzymatisch aktives Fragment von (a) oder (b) ist.

10. Verfahren nach den Ansprüchen 8 bis 9, **dadurch gekennzeichnet, dass** die chaotropen Agenzien chaotrope Salze sind.

11. Verfahren nach Anspruch 10, **dadurch gekennzeichnet, dass** die chaotropen Salze Guanidinhydrochlorid, Guanidinisothiocyanat und/oder Natriumiodid sind.

12. Verfahren nach den Ansprüchen 8 bis 11, **dadurch gekennzeichnet, dass** die Gesamtkonzentration der chaotropen Agenzien 0,1 mol/l bis 2,5 mol/l beträgt.

13. Verfahren nach den Ansprüchen 8 bis 12, **dadurch gekennzeichnet, dass** die Tenside Natriumdodecylsulfat, Brji40, Triton X-100 und/oder Tween20 sind.

14. Verfahren nach den Ansprüchen 8 bis 13, **dadurch gekennzeichnet, dass** die Gesamtmenge an Tensiden im Ansatz 0,1 - 2 Gew.-% beträgt.

15. Verfahren zur gezielten Isolierung von Nukleinsäure aus mikrobiellen Zellen, die in einer Mischprobe vorliegen, die höhere eukaryontische Zellen und/oder Gewebe umfasst, bei dem man
a) nur die höheren eukaryontischen Zellen und/oder Gewebe durch Zugabe eines oder mehrerer chaotroper Agenzien und eines oder mehrerer Tenside lysiert, wobei die mikrobiellen Zellen intakt bleiben;
b) die mikrobiellen Zellen von den lysierten höheren eukaryontischen Zellen und/oder Geweben abtrennt;
c) die mikrobiellen Zellen lysiert, sodass die Nukleinsäuren aus diesen freigesetzt werden;
d) die in Schritt c) freigesetzten Nukleinsäuren isoliert;
wobei man während oder nach Schritt a) eine DNA-abbauende Nuklease zusetzt, die keine RNAse-Aktivität aufweist, um die aus den höheren eukaryontischen Zellen freigesetzte DNA aus der Probe zu entfernen, **dadurch gekennzeichnet, dass** die DNA-abbauende Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

16. Verfahren nach Anspruch 15, **dadurch gekennzeichnet, dass** die DNA-abbauende Nuklease
a) die in SEQ ID NO:1 gezeigte Aminosäuresequenz aufweist;
b) eine Aminosäuresequenz aufweist, bei der bis zu 40% der Aminosäuren von der in SEQ ID NO:1 gezeigten Aminosäuresequenz abweichen; oder
c) ein enzymatisch aktives Fragment von (a) oder (b) ist.

17. Verfahren nach den Ansprüchen 15-16, **dadurch gekennzeichnet, dass** die chaotropen Agenzien chaotrope Salze sind.

18. Verfahren nach Anspruch 17, **dadurch gekennzeichnet, dass** die chaotropen Salze Guanidinhydrochlorid, Natriumiodid, Guanidinisothiocyanat, Natriumperchlorat und/oder Harnstoff sind.

19. Verfahren nach den Ansprüchen 15 bis 18, **dadurch gekennzeichnet, dass** die Tenside Natriumdodecylsulfat, Brij40, Triton X-100, und/oder Tween-20 sind.

20. Verfahren nach den Ansprüchen 15 bis 19, **dadurch gekennzeichnet, dass** die aus mikrobiellen Zellen zu isolierende Nukleinsäure DNA ist.

21. Verfahren nach den Ansprüchen 15 bis 20, **dadurch gekennzeichnet, dass** die aus mikrobiellen Zellen zu isolierende Nukleinsäure RNA ist.

22. Verfahren nach einem der Ansprüche 15 bis 21, **dadurch gekennzeichnet, dass** die Abtrennung in Schritt b) durch Zentrifugation erfolgt.

23. Verfahren nach einem der Ansprüche 15 bis 22, **dadurch gekennzeichnet, dass** die Abtrennung in Schritt b) durch Filtration erfolgt.

24. Verfahren nach einem der Ansprüche 15 bis 23, **dadurch gekennzeichnet, dass** die Lyse der mikrobiellen Zellen in Schritt c) durch mechanische Verfahren erfolgt.

25. Verfahren nach einem der Ansprüche 15 bis 23, **dadurch gekennzeichnet, dass** die Lyse der mikrobiellen Zellen in Schritt c) enzymatisch erfolgt.

26. Verfahren nach einem der Ansprüche 15 bis 23, **dadurch gekennzeichnet, dass** die Lyse der mikrobiellen Zellen in Schritt c) durch Zugabe eines oder mehrerer chaotroper Agenzien und eines oder mehrerer Tenside erfolgt.

27. Verfahren nach Anspruch 26, **dadurch gekennzeichnet, dass** die chaotropen Agenzien Guanidinhydrochlorid, Guanidinisothiocyanat und/oder Natriumiodid sind.

28. Verfahren nach Anspruch 26 oder 27, **dadurch gekennzeichnet, dass** die Tenside Natriumdodecylsulfat, Brji40, Triton X-100 und/oder Tween20 sind.

29. Verfahren nach einem der Ansprüche 15 bis 28, **dadurch gekennzeichnet, dass** die Mischprobe Blut, Blutprodukt, Urin, Stuhl, Sputum, Lavage, Punktat, Wundabstrich, Liquor und Bronchial-Alveolar-Lavage, Lymphe und/oder Sekret menschlichen oder tierischen Ursprungs und/oder Gewebe menschlichen oder tierischen Ursprungs oder Teile davon umfasst.

30. Verfahren nach Anspruch 29, **dadurch gekennzeichnet, dass** das Blutprodukt Thrombozytenkonzentrat oder Erythrozytenkonzentrat ist.

31. Verfahren nach den Ansprüchen 15 bis 29, **dadurch gekennzeichnet, dass** die höheren eukaryontischen Zellen Blutzellen oder Gewebezellen menschlichen oder tierischen Ursprungs sind.

32. Verfahren nach einem der Ansprüche 15 bis 28, **dadurch gekennzeichnet, dass** die höheren eukaryontischen Zellen Pflanzenzellen sind.

33. Verfahren nach Anspruch 32, **dadurch gekennzeichnet, dass** es sich bei den Pflanzenzellen um Material von Blatt, Stängel oder Wurzel der Pflanze handelt.

34. Verwendung eines Kits zur Durchführung eines Verfahrens nach den Ansprüchen 8 bis 14, wobei der Kit eine DNA-abbauende Nuklease, die keine RNAse-Aktivität aufweist und in der Lage ist, in Gegenwart von einem oder mehreren chaotropen Agenzien und einem oder mehreren Tensiden DNA abzubauen, und Puffer und Reagenzien für die Isolierung und/oder Aufreinigung von RNA umfasst, und wobei die Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

35. Verwendung eines Kits zur Durchführung eines Verfahrens nach den Ansprüchen 15 bis 33, wobei der Kit eine DNA-abbauende Nuklease, die keine RNAse-Aktivität aufweist und in der Lage ist, in Gegenwart von einem oder mehreren chaotropen Agenzien und einem oder mehreren Tensiden DNA abzubauen, und Puffer und Reagenzien für die Isolierung und/oder Aufreinigung von RNA umfasst, und wobei die Nuklease
a) die in SEQ ID NO:1, SEQ ID NO:2, SEQ ID NO:3 oder SEQ ID NO:4 gezeigte Aminosäuresequenz aufweist;
b) eine Aminosäuresequenz aufweist, bei der bis zu 40% der Aminosäuren von einer der Aminosäuresequenzen von (a) abweichen; oder
c) ein enzymatisch aktives Fragment von (a) oder (b) ist.

## Claims

1. Use of a DNA-degrading nuclease, which does not have RNAse activity, for the degradation of DNA in the presence of one or more chaotropic agents and one or more surfactants, wherein the DNA-degrading nuclease
(a) has the amino acid sequence set forth in SEQ ID NO:1, SEQ ID NO:2, SEQ ID NO:3 or SEQ ID NO:4;
(b) has an amino acid sequence, in which up to 40% of the amino acids differ from one of the amino acid sequences of (a); or
(c) is an enzymatically active fragment of (a) or (b).

2. Use according to claim 1, wherein the DNA-degrading nuclease
(a) has the amino acid sequence of SEQ ID NO:1;
(b) has an amino acid sequence, in which up to 40% of the amino acids differ from the amino acid sequence of SEQ ID NO:1; or
(c) is an enzymatically active fragment of (a) or (b).

3. Use according to claim 1 or 2, wherein the chaotropic agents are chaotropic salts.

4. Use according to claim 3, wherein the chaotropic salts are guanidine hydrochloride, guanidine isothiocyanate and/or sodium iodide.

5. Use according to any of claims 1-4, wherein the overall concentration of chaotropic agents is from 0.1 mol/l to 2.5 mol/l.

6. Use according to any of claims 1-5, wherein the surfactants are sodium dodecyl sulphate, Brji40, Triton X-100 and/or Tween20.

7. Use according to any of claims 1-6, wherein the overall amount of surfactants in the reaction is from 0.1 to 2% (w/w).

8. Method for the isolation and/or purification of RNA from mixtures comprising RNA and DNA, comprising
(a) degrading the DNA in the presence of one or more chaotropic agents and one or more surfactants using a DNA-degrading nuclease, which does not have RNAse activity; and
(b) isolating or purifying the RNA using appropriate methods,
wherein the DNA-degrading nuclease
(a) has the amino acid sequence set forth in SEQ ID NO:1, SEQ ID NO:2, SEQ ID NO:3 or SEQ ID NO:4;
(b) has an amino acid sequence, in which up to 40% of the amino acids differ from one of the amino acid sequences of (a); or
(c) is an enzymatically active fragment of (a) or (b).

9. Method according to claim 8, wherein the DNA-degrading nuclease
(a) has the amino acid sequence set forth in SEQ ID NO:1;
(b) has an amino acid sequence, in which up to 40% of the amino acids differ from the amino acid sequence of SEQ ID NO:1; or
(c) is an enzymatically active fragment of (a) or (b).

10. Method according to any of claims 8-9, wherein the chaotropic agents are chaotropic salts.

11. Use according to claim 10, wherein the chaotropic salts are guanidine hydrochloride, guanidine isothiocyanate and/or sodium iodide.

12. Use according to any of claims 8-11, wherein the overall concentration of chaotropic agents is from 0.1 mol/l to 2.5 mol/l.

13. Use according to any of claims 8-12, wherein the surfactants are sodium dodecyl sulphate, Brji40, Triton X-100 and/or Tween20.

14. Use according to any of claims 8-13, wherein the overall amount of surfactants in the reaction is from 0.1 to 2% (w/w).

15. Method for the targeted isolation of nucleic acids from microbial cells which are present in a mixed sample comprising higher eukaryotic cells and/or tissue, comprising
(a) lysing only the higher eukaryotic cells and/or tissue by the addition of one or more chaotropic agents and one or more surfactants, wherein the microbial cells remain intact;
(b) separating the microbial cells from the lysed higher eukaryotic cells and/or tissues;
(c) lysing the microbial cells to release the nucleic acids;
(d) isolating the nucleic acids released in c);
wherein a DNA-degrading nuclease which does not have RNAse activity is added during or after step a) to remove the DNA released from the higher eukaryotic cells from the sample, wherein the DNA-degrading nuclease
(a) has the amino acid sequence set forth in SEQ ID NO:1, SEQ ID NO:2, SEQ ID NO:3 or SEQ ID NO:4;
(b) has an amino acid sequence, in which up to 40% of the amino acids differ from one of the amino acid sequence of (a); or
(c) is an enzymatically active fragment of (a) or (b).

16. Method according to claim 15, wherein the DNA-degrading nuclease
(a) has the amino acid sequence of SEQ ID NO:1;
(b) has an amino acid sequence, in which up to 40% of the amino acids differ from the amino acid sequence of SEQ ID NO:1; or
(c) is an enzymatically active fragment of (a) or (b).

17. Method according to any of claims 15-16, wherein the chaotropic agents are chaotropic salts.

18. Method according to claim 17, wherein the chaotropic salts are guanidine hydrochloride, sodium iodide, guanidine isothiocyanate, sodium perchlorate and/or urea.

19. Method according to any of claims 15-18, wherein the surfactants are sodium dodecyl sulphate, Brji40, Triton X-100 and/or Tween20.

20. Method according to any of claims 15-19, wherein the nucleic acid to be isolated from the microbial cells is DNA.

21. Method according to any of claims 15-20, wherein the nucleic acid to be isolated from the microbial cells is RNA.

22. Method according to any of claims 15-21, wherein the separation in step b) is performed by centrifugation.

23. Method according to any of claims 15-22, wherein the separation in step b) is performed by filtration.

24. Method according to any of claims 15-23, wherein the lysis of the microbial cells in step c) is performed by mechanical methods.

25. Method according to any of claims 15-23, wherein the lysis of the microbial cells in step c) is performed enzymatically.

26. Method according to any of claims 15-23, wherein the lysis of the microbial cells in step c) is performed by adding one or more chaotropic agents and one or more surfactants.

27. Method according to claim 26, wherein the chaotropic agents are guanidine hydrochloride, guanidine isothiocyanate and/or sodium iodide.

28. Method according to any of claims 26 or 27, wherein the surfactants are sodium dodecyl sulphate, Brji40, Triton X-100 and/or Tween20.

29. Method according to any one of claims 15-28, wherein the mixed sample comprises blood, blood product, urine, stool, sputum, lavage, aspirate, wound smear, liquor and bronchoalveolar lavage, lymph and/or secretion of human or animal origin and/or tissue of human or animal origin or parts thereof.

30. Method according to claim 29, wherein the blood product is platelet concentrate or erythrocyte concentrate.

31. Method according to any of claims 15-29, wherein the higher eukaryotic cells are blood cells or tissue cells of human or animal origin.

32. Method according to any of claims 15-28, wherein the higher eukaryotic cells are plant cells.

33. Method according to claim 32, wherein the plant cells are material obtained from the leave, stipe or root of the plant.

34. Use of a kit for carrying out a method according to any of claims 8-14, wherein the kit comprises a DNA-degrading nuclease, which does not have RNAse activity and which is capable of degrading DNA in the presence of one or more chaotropic agents and one or more surfactants, and buffers and reagents for the isolation and/or purification of RNA, and wherein the nuclease
(a) has the amino acid sequence set forth in SEQ ID NO:1, SEQ ID NO:2, SEQ ID NO:3 or SEQ ID NO:4;
(b) has an amino acid sequence, in which up to 40% of the amino acids differ from one of the amino acid sequences of (a); or
(c) is an enzymatically active fragment of (a) or (b).

35. Use of a kit for carrying out a method according to any of claims 15-33, wherein the kit comprises a DNA-degrading nuclease, which does not have RNAse activity and which is capable of degrading DNA in the presence of one or more chaotropic agents and one or more surfactants, and buffers and reagents for the isolation and/or purification of RNA, and wherein the nuclease
(a) has the amino acid sequence set forth in SEQ ID NO:1, SEQ ID NO:2, SEQ ID NO:3 or SEQ ID NO:4;
(b) has an amino acid sequence, in which up to 40% of the amino acids differ from one of the amino acid sequences of (a); or
(c) is an enzymatically active fragment of (a) or (b).

## Revendications

1. Utilisation d'une nucléase dégradant l'ADN, qui ne présente pas d'activité d'ARNase, pour la décomposition de l'ADN en présence d'un ou de plusieurs agents chaotropes et d'un ou de plusieurs tensioactifs, **caractérisé en ce que** la nucléase dégradant l'ADN
a) présente la séquence d'acides aminés indiquée dans la SEQ ID N°1, SEQ ID N°2, SEQ ID N°3 ou SEQ ID N°4 ;
b) présente une séquence d'acides aminés dans laquelle jusqu'à 40 % des acides aminés diffèrent d'une des séquences d'acides aminés du (a) ; ou
c) est un fragment enzymatiquement actif du (a) ou (b).

2. Utilisation selon la revendication 1, **caractérisée en ce que** la nucléase dégradant l'ADN
a) présente la séquence d'acides aminés indiquée dans la SEQ ID N°1 ;
b) présente une séquence d'acides aminés dans laquelle jusqu'à 40 % des acides aminés diffèrent de la séquence d'acides aminés indiquée dans la SEQ ID N°1 ; ou
c) est un fragment enzymatiquement actif du (a) ou (b).

3. Utilisation selon les revendications 1 à 2, **caractérisée en ce que** les agents chaotropes sont des sels chaotropes.

4. Utilisation selon la revendication 3, **caractérisée en ce que** les sels chaotropes sont le chlorhydrate de guanidine, l'isothiocyanate de guanidine et/ou l'iodure de sodium.

5. Utilisation selon les revendications 1 à 4, **caractérisée en ce que** la concentration totale des agents chaotropes est de 0,1 mol/litre à 2,5 mol/litre.

6. Utilisation selon les revendications 1 à 5, **caractérisée en ce que** les tensioactifs sont le dodécylsulfate de sodium, Brij40, Triton X-100 et/ou Tween20.

7. Utilisation selon les revendications 1 à 6, **caractérisée en ce que** la quantité totale de tensioactifs dans la préparation est de 0,1 à 2 % en poids.

8. Procédé d'isolation et/ou de purification de l'ARN à partir de mélanges comprenant de l'ARN et de l'ADN, dans lequel on
a) décompose l'ADN en utilisant une nucléase dégradant l'ADN qui ne présente pas d'activité d'ARNase, en présence d'un ou de plusieurs agents chaotropes et d'un ou de plusieurs tensioactifs ; et
b) isole ou purifie l'ARN au moyen de procédés appropriés,
**caractérisé en ce que** la nucléase dégradant l'ADN
a) présente la séquence d'acides aminés indiquée dans la SEQ ID N°1, SEQ ID N°2, SEQ ID N°3 ou SEQ ID N°4 ;
b) présente une séquence d'acides aminés dans laquelle jusqu'à 40 % des acides aminés diffèrent d'une des séquences d'acides aminés du (a) ; ou
c) est un fragment enzymatiquement actif du (a) ou (b).

9. Procédé selon la revendication 8, **caractérisé en ce que** la nucléase dégradant l'ADN
a) présente la séquence d'acides aminés indiquée dans la SEQ ID N°1 ;
b) présente une séquence d'acides aminés dans laquelle jusqu'à 40 % des acides aminés diffèrent de la séquence d'acides aminés indiquée dans la SEQ ID N°1 ; ou
c) est un fragment enzymatiquement actif du (a) ou (b).

10. Procédé selon les revendications 8 à 9, **caractérisé en ce que** les agents chaotropes sont des sels chaotropes.

11. Procédé selon la revendication 10, **caractérisé en ce que** les sels chaotropes sont le chlorhydrate de guanidine, l'isothiocyanate de guanidine et/ou l'iodure de sodium.

12. Procédé selon les revendications 8 à 11, **caractérisé en ce que** la concentration totale des agents chaotropes est de 0,1 mol/litre à 2,5 mol/litre.

13. Procédé selon les revendications 8 à 12, **caractérisé en ce que** les tensioactifs sont le dodécylsulfate de sodium, Brij40, Triton X-100 et/ou Tween20.

14. Procédé selon les revendications 8 à 13, **caractérisé en ce que** la quantité totale de tensioactifs dans la préparation est de 0,1 à 2 % en poids

15. Procédé pour l'isolation ciblée d'acides nucléiques à partir de cellules microbiennes, qui sont présentes dans un échantillon mixte, comprenant des cellules et/ou des tissus eucaryotes supérieur(e)s, dans lequel on
a) lyse seulement les cellules et/ou les tissus eucaryotes supérieur(e)s par addition d'un ou de plusieurs agents chaotropes et d'un ou de plusieurs tensioactifs, les cellules microbiennes restant intactes ;
b) sépare les cellules microbiennes des cellules et/ou tissus eucaryotes supérieur(e)s lysé(e)s ;
c) lyse les cellules microbiennes de sorte que les acides nucléiques soient libérés de celles-ci ;
d) isole les acides nucléiques libérés à l'étape c) ;
dans lequel on ajoute pendant ou après l'étape a) une nucléase dégradant l'ADN qui ne présente pas d'activité d'ARNase pour retirer de l'échantillon l'ADN libéré des cellules eucaryotes supérieures, **caractérisé en ce que** la nucléase dégradant l'ADN
a) présente la séquence d'acides aminés indiquée dans la SEQ ID N°1, SEQ ID N°2, SEQ ID N°3 ou SEQ ID N°4 ;
b) présente une séquence d'acides aminés dans laquelle jusqu'à 40 % des acides aminés diffèrent d'une des séquences d'acides aminés du (a) ; ou
c) est un fragment enzymatiquement actif du (a) ou (b).

16. Procédé selon la revendication 15, **caractérisé en ce que** la nucléase dégradant l'ADN
a) présente la séquence d'acides aminés indiquée dans la SEQ ID N°1 ;
b) présente une séquence d'acides aminés dans laquelle jusqu'à 40 % des acides aminés diffèrent de la séquence d'acides aminés indiquée dans la SEQ ID N°1 ; ou
c) est un fragment enzymatiquement actif du (a) ou (b).

17. Procédé selon les revendications 15 - 16, **caractérisé en ce que** les agents chaotropes sont des sels chaotropes.

18. Procédé selon la revendication 17, **caractérisé en ce que** les sels chaotropes sont le chlorhydrate de guanidine, l'iodure de sodium, l'isothiocyanate de guanidine, le perchlorate de sodium et/ou l'urée.

19. Procédé selon les revendications 15 à 18, **caractérisé en ce que** les tensioactifs sont le dodécylsulfate de sodium, Brij40, Triton X-100 et/ou Tween20.

20. Procédé selon les revendications 15 à 19, **caractérisé en ce que** l'acide nucléique à isoler à partir des cellules microbiennes est l'ADN.

21. Procédé selon les revendications 15 à 20, **caractérisé en ce que** l'acide nucléique à isoler à partir des cellules microbiennes est l'ARN.

22. Procédé selon l'une des revendications 15 à 21, **caractérisé en ce que** la séparation à l'étape b) se fait par centrifugation.

23. Procédé selon l'une des revendications 15 à 22, **caractérisé en ce que** la séparation à l'étape b) se fait par filtration.

24. Procédé selon l'une des revendications 15 à 23, **caractérisé en ce que** la lyse des cellules microbiennes à l'étape c) se fait par des procédés mécaniques.

25. Procédé selon l'une des revendications 15 à 23, **caractérisé en ce que** la lyse des cellules microbiennes à l'étape c) se fait enzymatiquement.

26. Procédé selon l'une des revendications 15 à 23, **caractérisé en ce que** la lyse des cellules microbiennes à l'étape c) se fait par addition d'un ou de plusieurs agents chaotropes et d'un ou de plusieurs tensioactifs.

27. Procédé selon la revendication 26, **caractérisé en ce que** les agents chaotropes sont le chlorhydrate de guanidine, l'isothiocyanate de guanidine et/ou l'iodure de sodium.

28. Procédé selon les revendications 26 ou 27, **caractérisé en ce que** les tensioactifs sont le dodécylsulfate de sodium, Brij40, Triton X-100 et/ou Tween20.

29. Procédé selon l'une des revendications 15 à 28, **caractérisé en ce que** l'échantillon mixte comprend du sang, un produit sanguin, de l'urine, des matières fécales, de l'expectoration, un lavage, une ponction, un frottis de plaie, du liquide cérébrospinal et un lavage broncho-alvéolaire, de la lymphe et/ou une sécrétion d'origine humaine ou animale et/ou des tissus d'origine humaine ou animale ou des parties de ceux-ci.

30. Procédé selon la revendication 29, **caractérisé en ce que** le produit sanguin est un concentré de thrombocytes ou un concentré d'érythrocytes.

31. Procédé selon les revendications 15 à 29, **caractérisé en ce que** les cellules eucaryotes supérieures sont des cellules sanguines ou des cellules tissulaires d'origine humaine ou animale.

32. Procédé selon les revendications 15 à 28, **caractérisé en ce que** les cellules eucaryotes supérieures sont des cellules végétales.

33. Procédé selon la revendication 32, **caractérisé en ce que** les cellules végétales sont de la matière de feuilles, de tiges ou de racines de plante.

34. Utilisation d'un kit pour la réalisation d'un procédé selon les revendications 8 à 14, dans laquelle le kit comprend une nucléase dégradant l'ADN, qui ne présente pas d'activité d'ARNase et est en mesure de décomposer l'ADN en présence d'un ou de plusieurs agents chaotropes et d'un ou de plusieurs tensioactifs, et des tampons et des réactifs pour l'isolation et/ou la purification de l'ARN, et dans laquelle la nucléase
a) présente la séquence d'acides aminés indiquée dans la SEQ ID N°1, SEQ ID N°2, SEQ ID N°3 ou SEQ ID N°4 ;
b) présente une séquence d'acides aminés dans laquelle jusqu'à 40 % des acides aminés diffèrent d'une des séquences d'acides aminés du (a) ; ou
c) est un fragment enzymatiquement actif du (a) ou (b).

35. Utilisation d'un kit pour la réalisation d'un procédé selon les revendications 15 à 33, dans laquelle le kit comprend une nucléase dégradant l'ADN, qui ne présente pas d'activité d'ARNase et est en mesure de décomposer l'ADN en présence d'un ou de plusieurs agents chaotropes et d'un ou de plusieurs tensioactifs, et des tampons et des réactifs pour l'isolation et/ou la purification de l'ARN, et dans laquelle la nucléase
a) présente la séquence d'acides aminés indiquée dans la SEQ ID N°1, SEQ ID N°2, SEQ ID N°3 ou SEQ ID N°4 ;
b) présente une séquence d'acides aminés dans laquelle jusqu'à 40 % des acides aminés diffèrent d'une des séquences d'acides aminés du (a) ; ou
c) est un fragment enzymatiquement actif du (a) ou (b).
